# EUROPÄISCHE PATENTSCHRIFT

(11) **EP 1 633 873 B1**
(45) Veröffentlichungstag und Bekanntmachung des Hinweises auf die Patenterteilung: **14.07.2010**
(21) Anmeldenummer: 04738576.0
(22) Anmeldetag: 28.05.2004
(51) Int. Cl.: C12N 15/31, C12N 1/21, C07K 14/31, C07K 14/32, C12Q 1/68

(54) **BAKTERIEN MIT ERHÖHTER PROTEINSEKRETION, NUKLEOTIDSEQUENZEN CODIEREND FUR EIN SECA PROTEIN MIT ERHÖHTER PROTEINSEKRETION SOWIE VERFAHREN ZUR PRODUKTION VON PROTEINEN**
BACTERIA WITH INCREASED LEVELS OF PROTEIN SECRETION, NUCLEOTIDE SEQUENCES CODING FOR A SECA PROTEIN WITH INCREASED LEVELS OF PROTEIN SECRETION, AND METHOD FOR PRODUCING PROTEINS
BACTERIES PRESENTANT UN TAUX DE SECRETION PROTEIQUE AUGMENTE, SEQUENCES DE NUCLEOTIDES CODANT UNE PROTEINE SECA AVEC UN TAUX DE SECRETION PROTEIQUE AUGMENTE ET PROCEDE DE PRODUCTION DE PROTEINES

(30) Priorität: 02.06.2003 DE 10325026
(43) Veröffentlichungstag der Anmeldung: 15.03.2006
(73) Patentinhaber: Danisco US Inc., Palo Alto, CA 94304 (US)
(72) Erfinder: KÖBERLING, Oliver, 53019 Castelnuovo Berardenga (SI) (IT); FREUDL, Roland, 52353 Düren (DE)
(74) Vertreter: Wibbelmann, Jobst
(86) Internationale Anmeldenummer: PCT/DE2004/001107
(87) Internationale Veröffentlichungsnummer: WO 2004/108932

(56) Entgegenhaltungen:
- EP-A- 0 892 064
- MATSUMOTO GEN ET AL: "Genetic dissection of SecA: Suppressor mutations against the secY205 translocase defect" GENES TO CELLS, Bd. 5, Nr. 12, Dezember 2000 (2000-12), Seiten 991-999, XP002302227 ISSN: 1356-9597 in der Anmeldung erwähnt
- BAUD CATHERINE ET AL: "Allosteric communication between signal peptides and the SecA protein DEAD motor ATPase domain" JOURNAL OF BIOLOGICAL CHEMISTRY, Bd. 277, Nr. 16, 19. April 2002 (2002-04-19), Seiten 13724-13731, XP002302228 ISSN: 0021-9258
- KLEIN M ET AL: "Functional characterization of S. carnosus SecA protein in E. coli and B. subtilis secA mutant strains" FEMS MICROBIOLOGY LETTERS, AMSTERDAM, NL, Bd. 131, 1995, Seiten 271-277, XP002089426 ISSN: 0378-1097

### SEQUENZPROTOKOLL

<110> Forschungszentrum Jülich GmbH
<120> Bakterien mit erhöhter Proteinsekretion
<130> PT 1.2076
<140>
   <141>
<160> 4
<170> PatentIn Ver. 2.1
<210> 1
   <211> 2535
   <212> DNA
   <213> Staphylococcus carnosus
<400> 1
   atgggttttt taacaaaaat tgttgacggc aataagagag aaatcaaacg cctaagtaag 60
   caagctgaca aagtaatctc attagaagaa gaaatgtcaa ttcttactga tgaagaaatt 120
   agaaataaaa caaaagcatt ccaagaaaga ttgcaagcag aagaagatgt aagcaaacaa 180
   gataaaattt tagaagaaat attacctgaa gcatttgcgc ttgtccgtga aggagctaaa 240
   cgtgtattta atatgacacc ttatccagtt caaatcatgg gtggtatcgc cattcataat 300
   ggtgacattt cagaaatgag aacaggtgaa ggtaaaacat taactgcaac gatgccgact 360
   tatttaaacg ccttagcagc acgtggtgtg catgttatta cagtcaatga atacttggca 420
   agttctcaaa gagaagaaat ggccgagtta tataatttcc ttggtttatc agtcggattg 480
   aacttgaaca gcttatcaac agaacaaaag cgtgaagctt ataatgcaga tattacgtat 540
   agtacaaata atgaattagg cttcgactat ttacgcgata acatggtgaa ttattcagaa 600
   gaacgtgtta tgcgtccgct tcatttcgct atcattgatg aggtcgactc tattttaatc 660
   gatgaagcgc gtacaccatt gattatttca ggggaagctg aaaaatcaac atctctttat 720
   acacaagcaa atgttttcgc taaaatgtta aaagcagaag atgattataa ttatgatgaa 780
   aaaacaaaat cagtacaatt aacagatcaa ggtgctgata aagctgaacg tatgttcaag 840
   ttagataact tatatgattt gaaaaacgtt gatattatca cgcatatcaa tacagcatta 900
   cgtgctaact atacattgca acgcgatgta gattacatgg ttgtagatgg agaagtattg 960
   attgtcgacc aatttacagg tcgaacaatg ccaggtcgtc gattctctga aggacttcac 1020
   caagcgattg aggctaaaga aggggttcaa attcaaaatg aatctaaaac aatggcttct 1080
   atcacattcc aaaactactt ccgtatgtat aataaattag ccggtatgac aggtactgct 1140
   aaaacagagg aagaagaatt ccgtaacatt tataatatga cagttacaca aattccaacg 1200
   aaccgtcctg ttcaacgtga agatagacct gacttgattt tcatcagcca aaaaggcaag 1260
   ttcgatgctg ttgttgaaga tgttgttgaa aaacataaaa aaggccaacc aattctttta 1320
   ggtactgtag cggttgaaac aagtgaatac atttcacaac tattgaaaaa acgcggtgtg 1380
   cgtcatgatg tcttaaacgc taaaaaccat gaacgcgaag ctgaaatcgt atctacagca 1440
   ggtcaaaaag gtgcagtcac aatcgcaaca aacatggctg gtcgtggtac cgatattaaa 1500
   ttaggcgaag gtgttgaaga attaggcggc cttgctgtta ttggtacaga acgtcatgaa 1560
   tcacgccgta tcgatgatca gttgcgtggt cgttctggac gacaaggtga ccgcggagaa 1620
   agccgtttct atttatcatt acaagatgag ttgatggtac gtttcggttc tgaacgtctg 1680
   caaaaaatga tgggccgatt aggtatggat gactctacac cgattgaatc aaaaatggta 1740
   tctcgagctg ttgaatctgc acaaaaacgt gttgaaggta acaacttcga tgcacgtaaa 1800
   cgtatcttag aatacgatga agttttacgt aaacaacgtg aaatcattta tggtgaacgt 1860
   aataatatta tcgattcaga atcaagttct gaattagtca ttacaatgat acgctctaca 1920
   ttagatcgtg caatcagtta ttatgtaaat gaagaattgg aagaaattga ctatgcgccg 1980
   tttattaatt ttgtggaaga tgttttcttg cacgaaggtg aagtcaaaga agatgaaatc 2040
   aaaggtaaag gtaaagatcg tgaggatatt ttcgatacag tatgggctaa aattgaaaaa 2100
   gcttatgaag cacaaaaagc caatataccc gaccaattca atgaattcga acgtatgatt 2160
   ttattacgtt ctattgatgg aagatggaca gaccatatcg atacaatgga tcaattacgt 2220
   caaggtatcc atttacgttc atacggtcaa caaaacccac ttcgcgacta tcaaaatgaa 2280
   gggcaccaac tatttgatac aatgatggtc aatattgaag aagacgtcag caaatatatc 2340
   ttgaaatcaa ttatcacagt agatgatgat attgaacgtg ataaagcaaa agaatatcaa 2400
   ggacaacatg tatcagctga agatggaaaa gaaaaagtaa aaccgcaacc agttgttaaa 2460
   gataatcaca tcggaagaaa tgatccttgt ccatgcggca gcggtaaaaa gtataaaaat 2520
   tgctgcggta aatag                                                  2535
<210> 2
   <211> 844
   <212> PRT
   <213> Staphylococcus carnosus
<400> 2
   Met Gly Phe Leu Thr Lys Ile Val Asp Gly Asn Lys Arg Glu Ile Lys
   1               5                   10                  15
   Arg Leu Ser Lys Gln Ala Asp Lys Val Ile Ser Leu Glu Glu Glu Met
               20                  25                  30
   Ser Ile Leu Thr Asp Glu Glu Ile Arg Asn Lys Thr Lys Ala Phe Gln
           35                  40                  45
   Glu Arg Leu Gln Ala Glu Glu Asp Val Ser Lys Gln Asp Lys Ile Leu
       50                  55                  60
   Glu Glu Ile Leu Pro Glu Ala Phe Ala Leu Val Arg Glu Gly Ala Lys
   65                  70                  75                  80
   Arg Val Phe Asn Met Thr Pro Tyr Pro Val Gln Ile Met Gly Gly Ile
                   85                  90                  95
   Ala Ile His Asn Gly Asp Ile Ser Glu Met Arg Thr Gly Glu Gly Lys
               100                 105                 110
   Thr Leu Thr Ala Thr Met Pro Thr Tyr Leu Asn Ala Leu Ala Ala Arg
           115                 120                 125
   Gly Val His Val Ile Thr Val Asn Glu Tyr Leu Ala Ser Ser Gln Arg
   130 135 140
   Glu Glu Met Ala Glu Leu Tyr Asn Phe Leu Gly Leu Ser Val Gly Leu
   145 150 155 160
   Asn Leu Asn Ser Leu Ser Thr Glu Gln Lys Arg Glu Ala Tyr Asn Ala
   165 170 175
   Asp Ile Thr Tyr Ser Thr Asn Asn Glu Leu Gly Phe Asp Tyr Leu Arg
   180 185 190
   Asp Asn Met Val Asn Tyr Ser Glu Glu Arg Val Met Arg Pro Leu His
   195 200 205
   Phe Ala Ile Ile Asp Glu Val Asp Ser Ile Leu Ile Asp Glu Ala Arg
   210 215 220
   Thr Pro Leu Ile Ile Ser Gly Glu Ala Glu Lys Ser Thr Ser Leu Tyr
   225 230 235 240
   Thr Gln Ala Asn Val Phe Ala Lys Met Leu Lys Ala Glu Asp Asp Tyr
   245 250 255
   Asn Tyr Asp Glu Lys Thr Lys Ser Val Gln Leu Thr Asp Gln Gly Ala
   260 265 270
   Asp Lys Ala Glu Arg Met Phe Lys Leu Asp Asn Leu Tyr Asp Leu Lys
   275 280 285
   Asn Val Asp Ile Ile Thr His Ile Asn Thr Ala Leu Arg Ala Asn Tyr
   290 295 300
   Thr Leu Gln Arg Asp Val Asp Tyr Met Val Val Asp Gly Glu Val Leu
   305 310 315 320
   Ile Val Asp Gln Phe Thr Gly Arg Thr Met Pro Gly Arg Arg Phe Ser
   325 330 335
   Glu Gly Leu His Gln Ala Ile Glu Ala Lys Glu Gly Val Gln Ile Gln
   340 345 350
   Asn Glu Ser Lys Thr Met Ala Ser Ile Thr Phe Gln Asn Tyr Phe Arg
   355 360 365
   Met Tyr Asn Lys Leu Ala Gly Met Thr Gly Thr Ala Lys Thr Glu Glu
   370 375 380
   Glu Glu Phe Arg Asn Ile Tyr Asn Met Thr Val Thr Gln Ile Pro Thr
   385 390 395 400
   Asn Arg Pro Val Gln Arg Glu Asp Arg Pro Asp Leu Ile Phe Ile Ser
   405 410 415
   Gln Lys Gly Lys Phe Asp Ala Val Val Glu Asp Val Val Glu Lys His
   420 425 430
   Lys Lys Gly Gln Pro Ile Leu Leu Gly Thr Val Ala Val Glu Thr Ser
   435 440 445
   Glu Tyr Ile Ser Gln Leu Leu Lys Lys Arg Gly Val Arg His Asp Val
   450 455 460
   Leu Asn Ala Lys Asn His Glu Arg Glu Ala Glu Ile Val Ser Thr Ala
   465 470 475 480
   Gly Gln Lys Gly Ala Val Thr Ile Ala Thr Asn Met Ala Gly Arg Gly
   485 490 495
   Thr Asp Ile Lys Leu Gly Glu Gly Val Glu Glu Leu Gly Gly Leu Ala
   500 505 510
   Val Ile Gly Thr Glu Arg His Glu Ser Arg Arg Ile Asp Asp Gln Leu
   515 520 525
   Arg Gly Arg Ser Gly Arg Gln Gly Asp Arg Gly Glu Ser Arg Phe Tyr
   530 535 540
   Leu Ser Leu Gln Asp Glu Leu Met Val Arg Phe Gly Ser Glu Arg Leu
   545 550 555 560
   Gln Lys Met Met Gly Arg Leu Gly Met Asp Asp Ser Thr Pro Ile Glu
   565 570 575
   Ser Lys Met Val Ser Arg Ala Val Glu Ser Ala Gln Lys Arg Val Glu
   580 585 590
   Gly Asn Asn Phe Asp Ala Arg Lys Arg Ile Leu Glu Tyr Asp Glu Val
   595 600 605
   Leu Arg Lys Gln Arg Glu Ile Ile Tyr Gly Glu Arg Asn Asn Ile Ile
   610 615 620
   Asp Ser Glu Ser Ser Ser Glu Leu Val Ile Thr Met Ile Arg Ser Thr
   625 630 635 640
   Leu Asp Arg Ala Ile Ser Tyr Tyr Val Asn Glu Glu Leu Glu Glu Ile
                   645                 650                 655
   Asp Tyr Ala Pro Phe Ile Asn Phe Val Glu Asp Val Phe Leu His Glu
               660                 665                 670
   Gly Glu Val Lys Glu Asp Glu Ile Lys Gly Lys Gly Lys Asp Arg Glu
           675                 680                 685
   Asp Ile Phe Asp Thr Val Trp Ala Lys Ile Glu Lys Ala Tyr Glu Ala
       690                 695                 700
   Gln Lys Ala Asn Ile Pro Asp Gln Phe Asn Glu Phe Glu Arg Met Ile
   705                 710                 715                 720
   Leu Leu Arg Ser Ile Asp Gly Arg Trp Thr Asp His Ile Asp Thr Met
                   725                 730                 735
   Asp Gln Leu Arg Gln Gly Ile His Leu Arg Ser Tyr Gly Gln Gln Asn
               740                 745                 750
   Pro Leu Arg Asp Tyr Gln Asn Glu Gly His Gln Leu Phe Asp Thr Met
           755                 760                 765
   Met Val Asn Ile Glu Glu Asp Val Ser Lys Tyr Ile Leu Lys Ser Ile
       770                 775                 780
   Ile Thr Val Asp Asp Asp Ile Glu Arg Asp Lys Ala Lys Glu Tyr Gln
   785                 790                 795                 800
   Gly Gln His Val Ser Ala Glu Asp Gly Lys Glu Lys Val Lys Pro Gln
                   805                 810                 815
   Pro Val Val Lys Asp Asn His Ile Gly Arg Asn Asp Pro Cys Pro Cys
               820                 825                 830
   Gly Ser Gly Lys Lys Tyr Lys Asn Cys Cys Gly Lys
           835                 840
<210> 3
   <211> 2526
   <212> DNA
   <213> Bacillus subtilis
<400> 3
   atgcttggaa ttttaaataa aatgtttgat ccaacaaaac gtacgctgaa tagatacgaa 60
   aaaattgcta acgatattga tgcgattcgc ggagactatg aaaatctctc tgacgacgca 120
   ttgaaacata aaacaattga atttaaagag cgtcttgaaa aaggggcgac aacggatgat 180
   cttcttgttg aagctttcgc tgttgttcga gaagcttcac gccgcgtaac aggcatgttt 240
   ccgtttaaag tccagctcat ggggggcgtg gcgcttcatg acggaaatat agcggaaatg 300
   aaaacagggg aagggaaaac attaacgtct accctgcctg tttatttaaa tgcgttaacc 360
   ggtaaaggcg tacacgtcgt gactgtcaac gaatacttgg caagccgtga cgctgagcaa 420
   atggggaaaa ttttcgagtt tctcggtttg actgtcggtt tgaatttaaa ctcaatgtca 480
   aaagacgaaa aacgggaagc ttatgccgct gatattactt actccacaaa caacgagctt 540
   ggcttcgact atttgcgtga caatatggtt ctttataaag agcagatggt tcagcgcccg 600
   cttcattttg cggtaataga tgaagttgac tctattttaa ttgatgaagc aagaacaccg 660
   cttatcattt ctggacaagc tgcaaaatcc actaagctgt acgtacaggc aaatgctttt 720
   gtccgcacgt taaaagcgga gaaggattac acgtacgata tcaaaacaaa agctgtacag 780
   cttactgaag aaggaatgac gaaggcggaa aaagcattcg gcatcgataa cctctttgat 840
   gtgaagcatg tcgcgctcaa ccaccatatc aaccaggcct taaaagctca cgttgcgatg 900
   caaaaggacg ttgactatgt agtggaagac ggacaggttg ttattgttga ttccttcacg 960
   ggacgtctga tgaaaggccg ccgctacagt gaggggcttc accaagcgat tgaagcaaag 1020
   gaagggcttg agattcaaaa cgaaagcatg accttggcga cgattacgtt ccaaaactac 1080
   ttccgaatgt acgaaaaact tgccggtatg acgggtacag ctaagacaga ggaagaagaa 1140
   ttccgcaaca tctacaacat gcaggttgtc acgatcccta ccaacaggcc tgttgtccgt 1200
   gatgaccgcc cggatttaat ttaccgcacg atggaaggaa agtttaaggc agttgcggag 1260
   gatgtcgcac agcgttacat gacgggacag cctgttctag tcggtacggt tgccgttgaa 1320
   acatctgaat tgatttctaa gctgcttaaa aacaaaggaa ttccgcatca agtgttaaat 1380
   gccaaaaacc atgaacgtga agcgcagatc attgaagagg ccggccaaaa aggcgcagtt 1440
   acgattgcga ctaacatggc ggggcgcgga acggacatta agcttggcga aggtgtaaaa 1500
   gagcttggcg ggctcgctgt agtcggaaca gaacgacatg aatcacgccg gattgacaat 1560
   cagcttcgag gtcgttccgg acgtcaggga gacccgggga ttactcaatt ttatctttct 1620
   atggaagatg aattgatgcg cagattcgga gctgagcgga caatggcgat gcttgaccgc 1680
   ttcggcatgg acgactctac tccaatccaa agcaaaatgg tatctcgcgc ggttgaatcg 1740
   tctcaaaaac gcgtcgaagg caataacttc gattcgcgta aacagcttct gcaatatgat 1800
   gatgttctcc gccagcagcg tgaggtcatt tataagcagc gctttgaagt cattgactct 1860
   gaaaacctgc gtgaaatcgt tgaaaatatg atcaagtctt ctctcgaacg cgcaattgca 1920
   gcctatacgc caagagaaga gcttcctgag gagtggaagc ttgacggtct agttgatctt 1980
   atcaacacaa cttatcttga tgaaggtgca cttgagaaga gcgatatctt cggcaaagaa 2040
   ccggatgaaa tgcttgagct cattatggat cgcatcatca caaaatataa tgagaaggaa 2100
   gagcaattcg gcaaagagca aatgcgcgaa ttcgaaaaag ttatcgttct tcgtgccgtt 2160
   gattctaaat ggatggatca tattgatgcg atggatcagc tccgccaagg gattcacctt 2220
   cgtgcttacg cgcagacgaa cccgcttcgt gagtatcaaa tggaaggttt tgcgatgttt 2280
   gagcatatga ttgaatcaat tgaggacgaa gtcgcaaaat ttgtgatgaa agctgagatt 2340
   gaaaacaatc tggagcgtga agaggttgta caaggtcaaa caacagctca tcagccgcaa 2400
   gaaggcgacg ataacaaaaa agcaaagaaa gcaccggttc gcaaagtggt tgatatcgga 2460
   cgaaatgccc catgccactg cggaagcggg aaaaaatata aaaattgctg cggccgtact 2520
   gaatag 2526
<210> 4
   <211> 841
   <212> PRT
   <213> Bacillus subtilis
<400> 4
   Met Leu Gly Ile Leu Asn Lys Met Phe Asp Pro Thr Lys Arg Thr Leu
   1 5 10 15
   Asn Arg Tyr Glu Lys Ile Ala Asn Asp Ile Asp Ala Ile Arg Gly Asp
   20 25 30
   Tyr Glu Asn Leu Ser Asp Asp Ala Leu Lys His Lys Thr Ile Glu Phe
   35 40 45
   Lys Glu Arg Leu Glu Lys Gly Ala Thr Thr Asp Asp Leu Leu Val Glu
   50 55 60
   Ala Phe Ala Val Val Arg Glu Ala Ser Arg Arg Val Thr Gly Met Phe
   65 70 75 80
   Pro Phe Lys Val Gln Leu Met Gly Gly Val Ala Leu His Asp Gly Asn
   85 90 95
   Ile Ala Glu Met Lys Thr Gly Glu Gly Lys Thr Leu Thr Ser Thr Leu
   100 105 110
   Pro Val Tyr Leu Asn Ala Leu Thr Gly Lys Gly Val His Val Val Thr
   115 120 125
   Val Asn Glu Tyr Leu Ala Ser Arg Asp Ala Glu Gln Met Gly Lys Ile
   130 135 140
   Phe Glu Phe Leu Gly Leu Thr Val Gly Leu Asn Leu Asn Ser Met Ser
   145 150 155 160
   Lys Asp Glu Lys Arg Glu Ala Tyr Ala Ala Asp Ile Thr Tyr Ser Thr
   165 170 175
   Asn Asn Glu Leu Gly Phe Asp Tyr Leu Arg Asp Asn Met Val Leu Tyr
   180 185 190
   Lys Glu Gln Met Val Gln Arg Pro Leu His Phe Ala Val Ile Asp Glu
   195 200 205
   Val Asp Ser Ile Leu Ile Asp Glu Ala Arg Thr Pro Leu Ile Ile Ser
   210 215 220
   Gly Gln Ala Ala Lys Ser Thr Lys Leu Tyr Val Gln Ala Asn Ala Phe
   225 230 235 240
   Val Arg Thr Leu Lys Ala Glu Lys Asp Tyr Thr Tyr Asp Ile Lys Thr
   245 250 255
   Lys Ala Val Gln Leu Thr Glu Glu Gly Met Thr Lys Ala Glu Lys Ala
   260 265 270
   Phe Gly Ile Asp Asn Leu Phe Asp Val Lys His Val Ala Leu Asn His
   275 280 285
   His Ile Asn Gln Ala Leu Lys Ala His Val Ala Met Gln Lys Asp Val
   290 295 300
   Asp Tyr Val Val Glu Asp Gly Gln Val Val Ile Val Asp Ser Phe Thr
   305 310 315 320
   Gly Arg Leu Met Lys Gly Arg Arg Tyr Ser Glu Gly Leu His Gln Ala
   325 330 335
   Ile Glu Ala Lys Glu Gly Leu Glu Ile Gln Asn Glu Ser Met Thr Leu
   340 345 350
   Ala Thr Ile Thr Phe Gln Asn Tyr Phe Arg Met Tyr Glu Lys Leu Ala
   355 360 365
   Gly Met Thr Gly Thr Ala Lys Thr Glu Glu Glu Glu Phe Arg Asn Ile
   370 375 380
   Tyr Asn Met Gln Val Val Thr Ile Pro Thr Asn Arg Pro Val Val Arg
   385 390 395 400
   Asp Asp Arg Pro Asp Leu Ile Tyr Arg Thr Met Glu Gly Lys Phe Lys
   405 410 415
   Ala Val Ala Glu Asp Val Ala Gln Arg Tyr Met Thr Gly Gln Pro Val
   420 425 430
   Leu Val Gly Thr Val Ala Val Glu Thr Ser Glu Leu Ile Ser Lys Leu
   435 440 445
   Leu Lys Asn Lys Gly Ile Pro His Gln Val Leu Asn Ala Lys Asn His
   450 455 460
   Glu Arg Glu Ala Gln Ile Ile Glu Glu Ala Gly Gln Lys Gly Ala Val
   465 470 475 480
   Thr Ile Ala Thr Asn Met Ala Gly Arg Gly Thr Asp Ile Lys Leu Gly
   485 490 495
   Glu Gly Val Lys Glu Leu Gly Gly Leu Ala Val Val Gly Thr Glu Arg
   500 505 510
   His Glu Ser Arg Arg Ile Asp Asn Gln Leu Arg Gly Arg Ser Gly Arg
   515 520 525
   Gln Gly Asp Pro Gly Ile Thr Gln Phe Tyr Leu Ser Met Glu Asp Glu
   530 535 540
   Leu Met Arg Arg Phe Gly Ala Glu Arg Thr Met Ala Met Leu Asp Arg
   545 550 555 560
   Phe Gly Met Asp Asp Ser Thr Pro Ile Gln Ser Lys Met Val Ser Arg
   565 570 575
   Ala Val Glu Ser Ser Gln Lys Arg Val Glu Gly Asn Asn Phe Asp Ser
   580 585 590
   Arg Lys Gln Leu Leu Gln Tyr Asp Asp Val Leu Arg Gln Gln Arg Glu
   595 600 605
   Val Ile Tyr Lys Gln Arg Phe Glu Val Ile Asp Ser Glu Asn Leu Arg
   610 615 620
   Glu Ile Val Glu Asn Met Ile Lys Ser Ser Leu Glu Arg Ala Ile Ala
   625 630 635 640
   Ala Tyr Thr Pro Arg Glu Glu Leu Pro Glu Glu Trp Lys Leu Asp Gly
   645 650 655
   Leu Val Asp Leu Ile Asn Thr Thr Tyr Leu Asp Glu Gly Ala Leu Glu
   660 665 670
   Lys Ser Asp Ile Phe Gly Lys Glu Pro Asp Glu Met Leu Glu Leu Ile
   675 680 685
   Met Asp Arg Ile Ile Thr Lys Tyr Asn Glu Lys Glu Glu Gln Phe Gly
   690 695 700
   Lys Glu Gln Met Arg Glu Phe Glu Lys Val Ile Val Leu Arg Ala Val
   705 710 715 720
   Asp Ser Lys Trp Met Asp His Ile Asp Ala Met Asp Gln Leu Arg Gln
   725 730 735
   Gly Ile His Leu Arg Ala Tyr Ala Gln Thr Asn Pro Leu Arg Glu Tyr
   740 745 750
   Gln Met Glu Gly Phe Ala Met Phe Glu His Met Ile Glu Ser Ile Glu
   755 760 765
   Asp Glu Val Ala Lys Phe Val Met Lys Ala Glu Ile Glu Asn Asn Leu
   770 775 780
   Glu Arg Glu Glu Val Val Gln Gly Gln Thr Thr Ala His Gln Pro Gln
   785 790 795 800
   Glu Gly Asp Asp Asn Lys Lys Ala Lys Lys Ala Pro Val Arg Lys Val
   805 810 815
   Val Asp Ile Gly Arg Asn Ala Pro Cys His Cys Gly Ser Gly Lys Lys
   820 825 830
   Tyr Lys Asn Cys Cys Gly Arg Thr Glu
   835 840

## Beschreibung

Die vorliegende Erfindung betrifft SecA Proteine aus *Staphylococcus carnosus* und *Bacillus subtilis* mit erhöhter Sekretion für Proteine sowie Nukleinsäuren, welche diese SecA Proteine codieren. Die vorliegende Erfindung betrifft ferner eine Genstruktur oder einen Vektor, enthaltend wenigstens eine erfindungsgemäße Nukleinsäure sowie einen Mikroorganismus, enthaltend die erfindungsgemäße Nukleinsäure, die erfindungsgemäße Genstruktur oder den erfindungsgemäßen Vektor in replizierbarer Form. Des Weiteren betrifft die vorliegenden Erfindung ein Verfahren zur mikrobiellen Herstellung von Proteinen unter Verwendung eines genetisch veränderten Mikroorganismus, in den wenigstens eine erfindungsgemäße Nukleinsäure übertragen worden ist.

Gram-positive Bakterien (v.a. verschiedene *Bacillus-*Arten) können Proteine in großen Mengen in das umgebende Kulturmedium ausscheiden. Diese Fähigkeit wird schon seit langem für die industrielle Gewinnung einer Vielzahl von sekretierten Enzymen (wie z. B. Amylasen, Proteasen, Lipasen) eingesetzt. Die von der Industrie für diese Prozesse verwendeten Produktionsstämme werden durch wiederholte ungerichtete Mutagenese und anschließendes Screening auf eine gesteigerte Exoenzymproduktion hin optimiert, was zu Ausbeuten von mehreren Gramm Enzym pro Liter Kulturüberstand führen kann. Die Ursachen für die erhöhten Produktivitäten dieser Stämme sind meist nicht bekannt. Verschiedene Versuche, Gram-positive Bakterien als Wirtsorganismen für die sekretorische Gewinnung von heterologen Proteinen einzusetzen, lieferten oft nur ein enttäuschendes Ergebnis. Die erhaltenen Ausbeuten waren in den allermeisten Fällen deutlich geringer als die Mengen, die bei der Sekretion von homologen Exoproteinen erreicht werden können. Einer der Gründe hierfür ist der ineffiziente oder gar völlig ausbleibende Transport des heterologen Proteins über die Cytoplasmamembran [1-4].

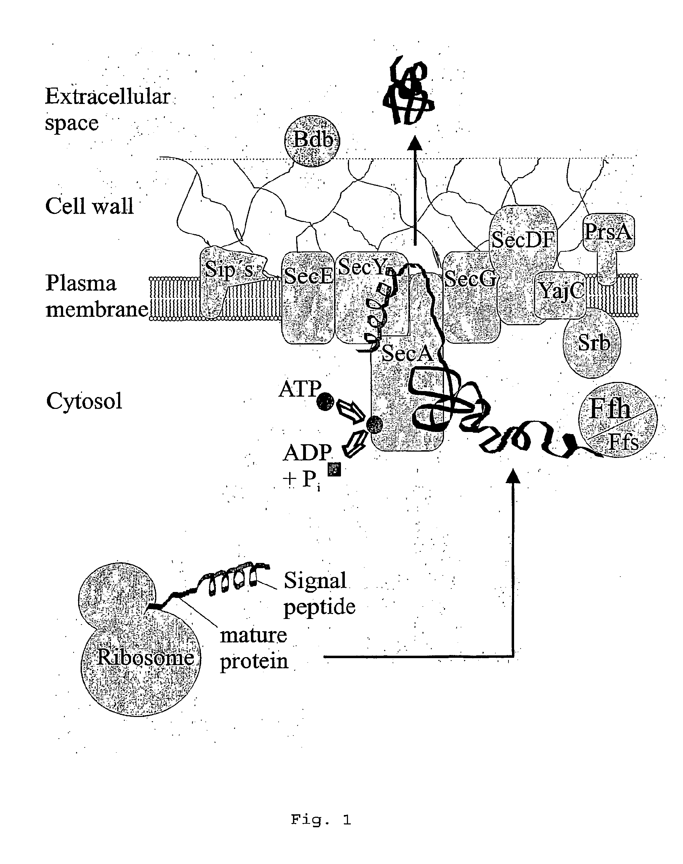

Der Transport von Proteinen über die bakterielle Plasmamembran wird durch die sogenannte Sec-Translokase (Fig.1) katalysiert. Diese besteht aus den membranintegralen Bestandteilen SecY, SecE, SecG, SecD, SecF und YajC sowie der zentralen Komponente SecA, die als sogenannte Translokations-ATPase die Energie der ATP-Bindung und -Hydrolyse an die Translokation der Polypeptidkette über die Membran koppelt [5,6].

Eine effiziente Initiation der Translokation eines sekretorischen Proteins über die bakterielle Cytoplasmamembran erfordert die Ausbildung eines funktionellen Komplexes aus mindestens dem SecA Protein, SecY und dem Exportprotein an der Membran. In Folge dieser funktionellen Wechselwirkungen, sowie dem Austausch von ADP gegen ATP an der Nukleotidbindestelle 1 (NBS I) von SecA, führt eine Konformationsänderung zur Membraninsertion von SecA, sowie der Aktivierung der SecA ATPase Aktivität und der Initiation der Translokation. Die ATP Hydrolyse an SecA wird durch einen zweifachen intramolekularen Mechanismus reguliert. Untersuchungen in *Escherichia coli* zeigten, dass im freien cytosolisch vorliegenden SecA die ATPase Aktivität durch Wechselwirkungen der regulatorischen Elemente IRA-1 und IRA-2 (intramolekularer Regulator der ATP Hydrolyse) mit der NBS 1 herabreguliert wird. Es wird angenommen, dass die auf Grund funktioneller Wechselwirkungen zwischen SecA, SecY und dem Exportprotein hervorgerufene Konformationsänderung von SecA zu einer räumlichen Entfernung vom IRA-1 und IRA-2 von der NBS 1 führt, wodurch die SecA ATPase aktiviert wird [7,8].

In der Literatur wurden verschiedene *Escherichia coli* SecA-Mutationen beschrieben, die eine Vielzahl von durch sec-Mutationen verursachte Defekte kompensieren können und daher die allgemeine Aktivität von SecA zu erhöhen scheinen [10].

Bei der sekretorischen Gewinnung von heterologen Proteinen mit gram-positiven Bakterien kann die Translokation einen limitierenden Schritt auf Grund der Qualitätskontrolle oder "Proofreading Aktivität" der Translokase darstellen. Dieser Kontrollmechanismus ist für die Zelle notwendig, um bezüglich der eigenen Proteine nur diejenigen in den Sekretionsweg einzuschleusen, die für den Export aus dem Cytosol bestimmt sind. Hinsichtlich heterologer Proteine, die nicht optimal an den fremden Exportapparat angepasst sind, kann diese Qualitätskontrolle jedoch einen wesentlichen limitierenden Schritt darstellen. Sehr wahrscheinlich ist die Fähigkeit der heterologen Exportproteine zur Aktivierung der Translokations-ATPase-Aktivität des SecA Proteins entscheidend dafür, ob und mit welcher Effizienz ein Membrantransport des heterologen Exportproteins stattfindet bzw. ob und in welchem Ausmaß eine Zurückweisung des heterologen Exportproteins durch die Qualitätskontrolle der Sec-Translokase erfolgt.

Es ist daher Aufgabe der Erfindung, Nukleotidsequenzen, Proteinsequenzen, Bakterien sowie ein Verfahren bereit zu stellen, mit denen eine gegenüber bisher bekannten mikrobiologischen Prozessen verbesserte sekretorische Gewinnung von Proteinen möglich wird.

Diese Aufgabe wird durch die SecA Proteine aus *Staphylococcus carnosus* und *Bacillus subtilis* gemäß den Ansprüchen 1 und 2, die Nukleinsäuren gemäß den Ansprüchen 3 und 4, die Genstruktur gemäß Anspruch 5, den Vektor gemäß Anspruch 6, den Mikroorganismus gemäß einem der Ansprüche 7 bis 11 und dem Verfahren zur mikrobiellen Herstellung von Proteinen gemäß einem der Ansprüche 12 bis 15 gelöst.

Mit den erfindungsgemäßen Nukleinsäuren sowie Polypeptiden ist es nunmehr möglich, Proteine, die bisher nur in geringer Menge oder gar nicht von den Mikroorganismen exportiert wurden, sekretorisch mit Mikroorganismen zu gewinnen bzw. mit einer erhöhten Effizienz herzustellen. Die erfindungsgemäßen Nukleinsäuren codieren gegenüber natürlich vorkommenden oder gentechnisch nicht veränderten Nukleinsäuren für ein Translokations-ATPase Protein, im folgenden mit SecA bezeichnet, welches eine erhöhte Sekretion für Proteine bewirkt bzw. aufweist. Unter der Bezeichnung "erhöhte Sekretion" wird eine im Vergleich zu Wild Typ organismen erhöhte oder auch zum ersten Mal mögliche Proteinsekretion verstanden. Weiterhin ist es möglich, Mikroorganismen und Verfahren bereitzustellen, mit denen eine Sekretion und Produktion von Proteinen mit gegenüber bisher bekannten mikrobiellen Verfahren höheren Ausbeuten bzw. zum ersten Mal möglich ist.

Ein ester Gegenstand der Erfindung ist dem nach ein SecA Protein (Translokations-ATPase), mit einer Aminosäuresequenz, welches gegenüber der Wild Typ SecA Aminosäuresequenz mindestens eine Veränderung der Aminosäuresequenz aufweist , wodurch ein SecA mit erhöhter Sekretion für Proteine ausgebildet wird.

In überraschender Weise wurde festgestellt, dass bereits eine Veränderung (= Austausch) einer Aminosäure der Aminosäuresequenz des SecA Proteins zu einer erhöhten Sekretion für Proteine bzw. zu einer erstmals möglichen Sekretion von Proteinen führt. Mehrere ausgetauschte Aminosäuren, beispielsweise 2 bis 7, können jedoch ebenfalls die erhöhte Sekretion für Proteine bewirken.

Veränderungen im Bereich der Aminosäuresequenzen, die für die Ausbildung der regulatorischen Elemente IRA-1/IRA-2 verantwortlich sind oder eine modifizierte Form dieser Polypeptidsequenzen oder Isoformen davon, erwiesen sich als vorteilhaft für das SecA mit erhöhter Sekretion für Proteine, wobei hier der Begriff "Bereich" nicht nur Veränderungen umfassen soll, die genau in den Aminosäurepositionen liegen, die für die Ausbildung der IRA-1/IRA-2 verantwortlich sind, sondern auch Veränderungen, die beispielsweise 250 bis 300 Aminosäuren vor oder hinter den jeweiligen Aminosäurepositionen für IRA-1 bzw. IRA-2 liegen.

Es konnte sowohl durch eine einzige Veränderung als auch durch mehrere Veränderungen im Bereich der Aminosäuresequenz, die für die Ausbildung von IRA-1 bzw. IRA-2 verantwortlich ist, sowie durch Kombinationen von Veränderungen in diesen Bereichen, ein SecA mit erhöhter Sekretion für Proteine erhalten werden.

Die erfindungsgemäßen Polypeptide zeichnen sich dadurch aus, dass sie aus gram-positiven Bakterien der Art Bacillus subtilis oder Staphylococcus carnosus stammen. Beispiele für in Stammkulturen bei der DSMZ (Deutsche Sammlung von Mikroorganismen und Zellkulturen GmbH, Braunschweig) erhältliche Bakterien sind z. B. Bacillus subtilis 168 bzw. Staphylococcus carnosus TM 300. Die vorliegende Erfindung wird durch die Angabe der zuvor genannten Bakterienstämme näher charakterisiert, die jedoch nicht limitierend wirkt.

Bei Staphylococcus carnosus erstrecken sich die Aminosäuresequenzen, die das IRA-1 im SecA ausbilden auf Position 721 bis 772 und für IRA-2 auf Position 448 bis 567.

Bei Bacillus subtilis erstrecken sich die Aminosäuresequenzen, die das IRA-1 im SecA ausbilden auf die Position 716 bis 767 und für IRA-2 auf die Position 442 bis 561.

Bei dem aus Staphylococcus carnosus isolierten SecA Protein erwies sich mindestens eine Veränderung im Bereich der Aminosäuren von Position 198 bis 772 oder eine modifizierte Form dieser Polypeptidsequenzen oder Isoformen davon, als vorteilhaft.

Bei dem aus Bacillus subtilis isolierten SecA Protein erwies sich mindestens eine Veränderung im Bereich der Aminosäuren von Position 442 bis 767 oder eine modifizierte Form dieser Polypeptidsequenzen oder Isoformen davon, als vorteilhaft.

Beschrieben hierin wird zudem ein *SecA* mit erhöhter Sekretion für Proteine oder ein Teil davon enthaltend eine Aminosäuresequenz gemäß SEQ ID No 2, welche mindestens eine Veränderung aus der Gruppe der Aminosäuren in Position 198, 470, 474, 493, 537, 665 und/oder 734 aufweist oder eine modifizierte Form dieser Polypeptidsequenzen oder Isoformen davon.

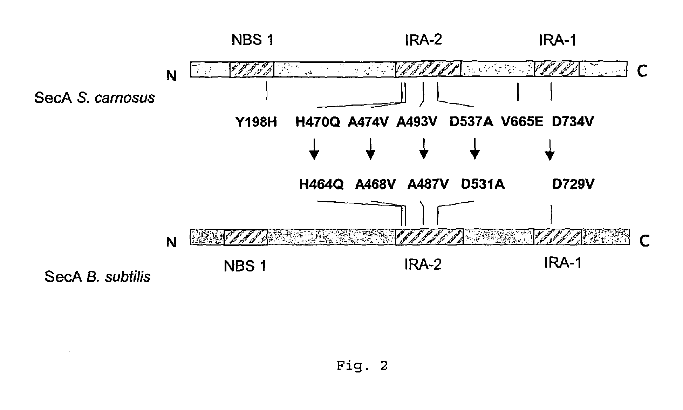

Folgende Aminosäureaustausche im SecA von Staphylococcus carnosus erwiesen (s. Fig. 2) sich beispielsweise als erfolgreich:
Position 198: Tyrosin zu Histidin
Position 470: Histidin zu Glutamin
Position 474: Alanin zu Valin
Position 493: Alanin zu Valin
Position 537: Asparaginsäure zu Alanin
Position 665: Valin zu Glutaminsäure
Position 734: Asparaginsäure zu Valin

Beschrieben hierin wird ebenso ein *SecA* mit erhöhter Sekretion für Proteine mit einer Aminosäuresequenz gemäß der SEQ ID No 4, welche mindestens eine Veränderung aus der Gruppe der Aminosäuren in Position 464, 468, 487, 531 und/oder 729 aufweist oder eine modifizierte Form dieser Polypeptidsequenzen oder Isoformen davon.

Folgende Aminosäureaustausche im SecA von Bacillus subtilis erwiesen (s. Fig. 2) sich beispielsweise als erfolgreich:
Position 464: Histidin zu Glutamin
Position 468: Alanin zu Valin
Position 487: Alanin zu Valin
Position 531: Asparaginsäure zu Alanin
Position 729: Asparaginsäure zu Valin

Unter Isoformen sind Proteine mit gleicher oder vergleichbarer Wirkungsspezifität zu verstehen, die jedoch eine unterschiedliche Primärstruktur aufweisen.

Unter modifizierten Formen sind Proteinne zu verstehen, bei denen Änderungen in der Sequenz, beispielsweise am C- oder N-Terminus des Polypeptids oder im Bereich konservierter Aminosäuren vorliegen, ohne jedoch die Funktion der erhöhten Proteinsekretion zu beeinträchtigen. Diese Veränderungen können in Form von Aminosäureaustauschen nach an sich bekannten Methoden vorgenommen werden.

In überraschender Weise wurde festgestellt, dass bereits eine Mutation in einem Nukleotid des *secA* Gens zu einer erhöhten Sekretion für Proteine bzw. zu einer erstmals möglichen Sekretion von Proteinen führt. Mehrere Mutationen, beispielsweise 2 bis 7, können jedoch ebenfalls die erhöhte Sekretion der Proteine bewirken. Mindestens eine Mutation in Nukleotidbereichen, die für die regulatorischen Elemente IRA-1 und/oder IRA-2 der SecA-Proteine codieren oder ein Allel, Homolog oder Derivat dieser Nukleotidsequenzen oder mit diesen hybridisierende Nukleotidsequenzen, erwies sich als vorteilhaft für die Expression eines SecA mit erhöhter Sekretion für Proteine, wobei unter dem Begriff "Bereich" nicht nur Mutationen umfasst sein sollen, die genau in den für die IRA-1/IRA-2 codierenden Bereichen liegen, sondern auch Mutationen umfasst sein sollen, die beispielsweise 750 bis 900 Nukleotide vor oder hinter den jeweils für IRA-1/IRA-2 codierenden Bereichen liegen.

Es konnte sowohl durch eine einzige Mutation als auch durch mehrere Mutationen im Bereich der für IRA-1 bzw. IRA-2 codierenden Nukleotidsequenzen sowie Kombination von Mutationen in diesen Bereichen, ein erhöhter Export von Proteinen bewirkt werden.

Die erfindungsgemäßen Nukleinsäuren zeichnen sich dadurch aus, dass sie aus den gram-positiven Bakterien Bacillus subtilis oder Staphylococus carnosus isoliert sind.

Bei Staphylococcus carnosus erstreckt sich der Nukleotidbereich, der für IRA-1 codiert, auf die Nukleotide von Position 2161 bis 2316 und für IRA-2 von Position 1342 bis 1701 (s.a. SEQ ID No 1).

Bei Bacillus subtilis erstreckt sich der Nukleotidbereich, der für IRA-1 codiert, auf die Nukleotide von Position 2146 bis 2301 und für IRA-2 von Position 1324 bis 1683 (s.a. SEQ ID No 3).

Bei den aus Staphylococcus carnosus isolierten Nukleinsäuren erwies sich mindestens eine Mutation im Bereich der Nukleotide von Position 592 bis 2210 oder ein Allel, Homolog oder Derivat dieser Nukleotidsequenzen oder mit diesen hybridisierende Nukleotidsequenzen, als vorteilhaft.

Bei den aus Bacillus subtilis isolierten Nukleinsäuren erwies sich mindestens eine Mutation im Bereich der Nukleotide von Position 1392 bis 2186 oder ein Allel, Homolog oder Derivat dieser Nukleotidsequenzen oder mit diesen hybridisierende Nukleotidsequenzen, als vorteilhaft.

Besonders vorteilhaft erwies sich eine Nukleinsäure enthaltend ein Gen *secA* gemäß SEQ ID No 1, welche mindestens eine Mutation im Gen aus der Gruppe der Nukleotide in Position 592, 1410, 1421, 1478, 1610, 1994 und/oder 2210 aufweist oder ein Allel, Homolog oder Derivat dieser Nukleotidsequenzen oder mit diesen hybridisierende Nukleotidsequenzen.

Folgende Mutationen erwiesen sich im *secA*-Gen von Staphylococcos carnosus als erfolgreich:
Position 592: T zu C
Position 1410: T zu A
Position 1421: C zu T
Position 1478: C zu T
Position 1610: A zu C
Position 1994: T zu A
Position 2210: A zu T

Weiterhin besonders vorteilhaft erwies sich eine Nukleinsäure, enthaltend ein *secA* Gen gemäß SEQ ID.No 3, welche mindestens eine Mutation aus der Gruppe der Nukleotide in Position 1392, 1403, 1404, 1460, 1461, 1592 und/oder 2186 aufweist oder ein Allel, Homolog oder Derivat dieser Nukleotidsequenzen oder mit diesen hybridisierende Nukleotidsequenzen.

Folgende Mutationen erwiesen sich im *secA*-Gen von Ba*cillus subtilis* als erfolgreich:
Position 1392: T zu A
Position 1403: C zu T
Position 1404: G zu T
Position 1460: C zu T
Position 1461: G zu T
Position 1592: A zu C
Position 2186: A zu T

Im Gegensatz zu den Mutationen in Position 1392, 1592 und 2186 führen die beiden Mutationen der Nukleotide in Position 1403 und 1404 bzw. 1460 und 1461 auf Proteinebene jeweils zum Austausch nur einer Aminosäure. Mit den beschriebenen Nukleinsäuren können Proteine, die in Gegenwart eines unveränderten SecA nur in geringem Maße bzw. gar nicht exportiert werden, effizient aus den Zellen exportiert werden. Die beschriebenen Nukleinsäuren, die für ein SecA mit erhöhter Sekretion für Proteine codieren, bewirken einen veränderten Kontrollmechanismus des SecA für die Proteine, die aus der Zelle ausgeschleust werden. Insbesondere heterologe Proteine, die nicht optimal an den Exportapparat der Wirtszelle angepasst sind, können nunmehr mit Hilfe der beschriebenen *secA* Sequenz besonders erfolgreich exportiert werden. Aber auch der Export von homologen Proteinen kann mit Hilfe der veränderten *secA* Sequenz in vorteilhafter Weise verbessert werden.

Unter einer Nukleinsäure oder einem Nukleinsäurefragment ist ein Polymer aus RNA oder DNA zu verstehen, das einzel- oder doppelsträngig sein kann und optional natürliche, chemisch synthetisierte, modifizierte oder artifizielle Nukleotide enthalten kann. Der Begriff DNA-Polymer schließt hierbei auch genomische DNA, cDNA oder Mischungen davon ein.

Unter Allelen sind äquivalente Nukleotidsequenzen zu verstehen, die für SecA Proteine mit erhöhter Sekretion für Proteine codieren. Äquivalente Sequenzen sind solche Sequenzen, welche trotz abweichender Nukleotidsequenz, beispielsweise durch die Degenerierung des genetischen Codes bedingt, noch für das SecA Protein mit der gewünschten erhöhten Sekretion für Proteine codieren. Äquivalente Nukleotidsequenzen umfassen somit natürlich vorkommende Varianten der hierin beschriebenen Sequenzen sowie künstliche, z. B. durch chemische Synthese erhaltene und gegebenenfalls an den Kodon-Gebrauch des Wirtsorganismus angepasste, Nukleotidsequenzen.

Unter äquivalenten Nukleotidsequenzen sollen auch Sequenzen mit Mutationen verstanden werden, insbesondre natürliche oder künstliche einer ursprünglich isolierten Sequenz, welche weiterhin für ein SecA Protein mit erhöhter Sekretion für Proteine codieren.

Mutationen der Äquivalente umfassen Substitutionen, Additionen, Deletionen, Vertauschungen oder Insertionen eines oder mehrerer Nukleotidreste. Inbegriffen sind hier auch sogenannte Sinnmutationen, die auf Proteinebene beispielsweise zum Austausch konservierter Aminosäuren führen können, welche aber zu keiner grundsätzlichen Veränderung der erhöhten Sekretion für Proteine des erfindungsgemäßen *SecA* Proteins führen und somit funktionsneutral sind. Dies beinhaltet auch Veränderungen der Nukleotidsequenz, die auf Proteinebene den C-Terminus oder N-Terminus eines Proteins betreffen, ohne jedoch die Funktion des Proteins wesentlich zu beeinträchtigen.

Unter homologen Sequenzen sind solche zu verstehen, die zu den Nukleotidequenzen komplementär sind und/oder mit diesen hybridisieren. Der Begriff hybridisierende Sequenzen schließt substanziell ähnliche Nukleotidsequenzen aus der Gruppe von DNA oder RNA ein, die unter an sich bekannten stringenten Bedingungen eine spezifische Wechselwirkung (Bindung) mit den zuvor genannten Nukleotidsequenzen eingehen. Hierzu zählen auch kurze Nukleotidsequenzen mit einer Länge von beispielsweise 10 bis 30, bevorzugt 12 bis 15 Nukleotiden. Dies umfasst u.a. auch sogenannte Primer oder Sonden.

Prinzipiell können Gene durch an sich bekannte Methoden, wie beispielsweise die Polymerase-Ketten-Reaktion (PCR) mit Hilfe von kurzen, synthetischen Nukleotidsequenzen (Primern) amplifiziert und anschließend isoliert werden. Die Herstellung der verwendeten Primer erfolgt im Allgemeinen anhand bekannter Gensequenzen aufgrund bestehender Homologien in konservierten Bereichen der Gene und/oder unter Berücksichtigung des GC-Gehalts der DNA des zu untersuchenden Mikroorganismus.

Eine weitere Vorgehensweise zur Isolierung von codierenden Nukleotidsequenzen ist die Komplementation von sogenannten Defekt-Mutanten, die zumindest phänotypisch einen Funktionsverlust in der Aktivität des zu untersuchenden Gens oder entsprechenden Proteins aufweisen. Unter einer Komplementation ist die Aufhebung des Gendefektes der Mutante und weitgehende Wiederherstellung des ursprünglichen Erscheinungsbildes vor der Mutagenese zu verstehen, die durch die Einbringung funktioneller Gene oder Genfragmente erreicht wird.

Ein klassisches Mutagenese-Verfahren zur Herstellung von Defektmutanten ist beispielsweise die Behandlung der Bakterienzellen mit Chemikalien wie z. B. N-Methyl-N-Nitro-N-Nitrosoguanidin oder UV-Bestrahlung. Derartige Verfahren zur Mutationsauslösung sind allgemein bekannt und können unter anderem bei Miller (A Short Course in Bacterial Genetics, A Laboratory Manual and Handbook for Escherichia coli and Related Bacteria (Cold Spring Harbor Laboratory Press, 1992)) oder im Handbuch "Manual of Methods for General Bacteriology" der American Society for Bacteriology (Washington D.C., USA, 1981) nachgelesen werden.

Ein weiterer Gegenstand der Erfindung ist eine Genstruktur enthaltend wenigstens eine erfindungsgemäße Nukleinsäure sowie mit diesen operativ verknüpfte regulatorische Sequenzen, welche die Expression der codierenden Sequenzen in der Wirtszelle steuern. Entsprechende Genstrukturen können beispielsweise Chromosomen, Plasmide, Vektoren, Phagen oder andere, nicht zirkulär geschlossene, Nukleotidsequenzen sein.

Die vorliegende Erfindung betrifft ferner einen Vektor enthaltend eine erfindungsgemäße Nukleinsäure , mit dieser operativ verknüpfte regulative Nukleotidsequenzen sowie zusätzliche Nukleotidsequenzen zur Selektion transformierter Wirtszellen, für die Replikation innerhalb der Wirtszelle oder zur Integration in das entsprechende Wirtszell-Genom.

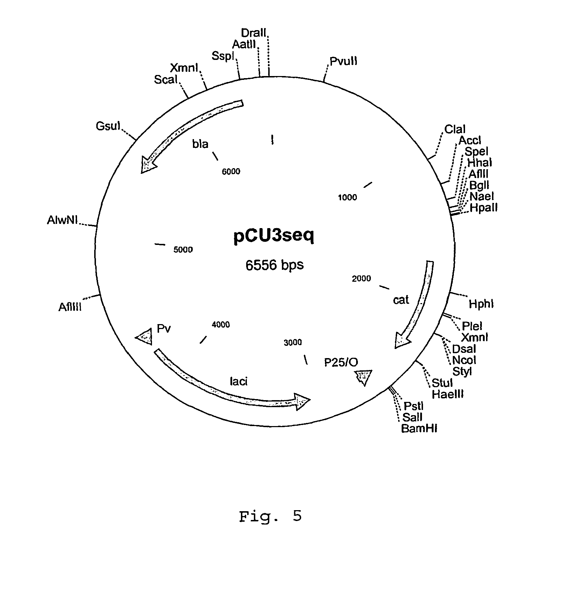

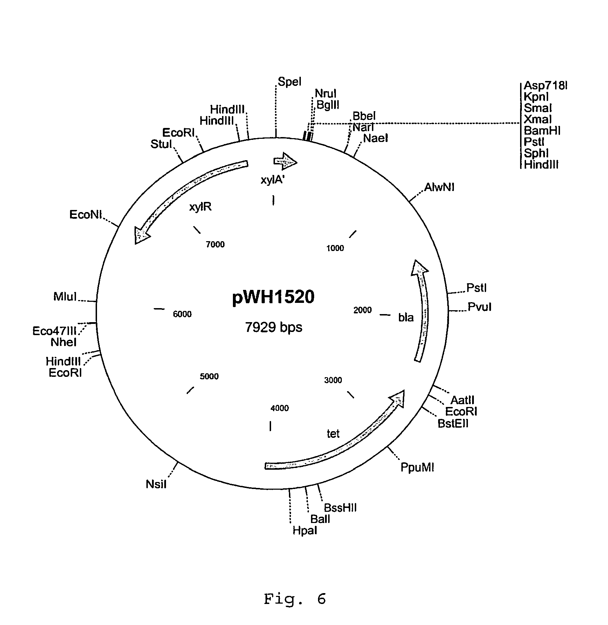

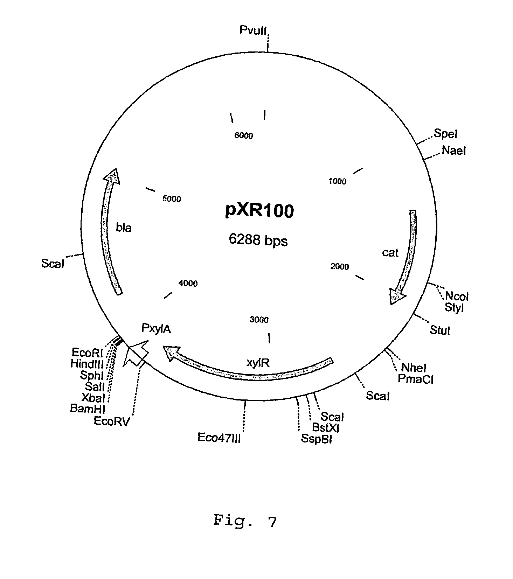

Als Vektoren eignen sich solche, die in Bakterien repliziert werden wie z. B. pWH1520 [17] pCU3 oder pXR100 (s, a. Figur 5, 6 bzw. 7). Andere Plasmidvektoren können in gleicher Weise verwendet werden. Diese Aufzählung ist für die vorliegende Erfindung jedoch nicht limitierend.

Unter Ausnutzung der hierin beschriebenen Nukleinsäuresequenzen können entsprechende Sonden oder auch Primer synthetisiert und dazu verwendet werden, beispielsweise mit Hilfe der PCR-Technik analoge Gene aus anderen Mikroorganismen, bevorzugt gram-positiven Bakterien zu amplifizieren und isolieren.

Beschrieben hierin wird daher auch eine Sonde zur Identifizierung und/oder Isolierung von Genen, codierend für am Export von Proteinen beteiligte Proteine, wobei diese Sonde ausgehend von den Nukleinsäuresequenzen der zuvor beschriebenen Art hergestellt wird und eine zur Detektion geeignete Markierung enthält. Bei der Sonde kann es sich um einen Teilausschnitt der hierin beschriebenen Sequenz, beispielsweise aus einem konservierten Bereich handeln, der z. B. eine Länge von 10 bis 30 oder bevorzugt 12 bis 15 Nukleotiden aufweist und unter stringenten Bedingungen spezifisch mit homologen Nukleotidsequenzen hybridisieren kann. Geeignete Markierungen sind aus der Literatur zahlreich bekannt. Anleitungen hierzu findet der Fachmann unter anderem beispielsweise im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994) oder beispielsweise im Handbuch "The DIG System Users Guide for Filter Hybridization" der Firma Roche Diagnostics (Mannheim, Deutschland) und bei Liebl et al. (International Journal of Systematic Bacteriology (1991) 41: 255-260).

Beschrieben hierin ist ferner die Übertragung wenigstens einer der beschriebenen Nukleinsäuresequenzen oder eines Teils davon, codierend für ein SecA mit erhöhter Sekretion für Proteine, ein Allel, Homolog oder Derivat davon in ein Wirtssystem. Dies schließt auch die Übertragung einer erfindungsgemäßen Genstruktur in ein Wirtssystem ein. Diese Übertragung von DNA in eine Wirtszelle erfolgt nach gentechnischen Methoden. Als bevorzugtes Verfahren sei hier die Transformation und besonders bevorzugt die Übertragung von DNA durch Elektroporation genannt.

Als besonders geeignet haben sich gram-positive Wirtsorganismen erwiesen. Ein aus einer erfolgreich durchgeführten Nukleinsäureübertragung resultierender transformierter Mikroorganismus unterscheidet sich von dementsprechend nicht transformierten Mikroorganismus dadurch, dass er Nukleinsäuren der hierin beschriebenen Art enthält und entsprechend zur Ausprägung bringen kann. Stellvertretend für ein geeignetes Wirtssystem seien Organismen der Gattung Bacillus oder Staphylococcus und bevorzugt der Art Bacillus subtilis, Bacillus licheniformis, Bacillus amyloliquefaciens bzw. Staphylococcus carnosus genannt. Als Kulturmedium ist je nach Anforderungen ein Komplexmedium wie z. B. LB Medium (T. Maniatis, E.F. Fritsch und J. Sambrook, Molecular Clonin: A Laboratory Manual, Cold Spring Harbor Laboratury, Cold Spring Harbor, NY 1989)) oder auch ein Mineralsalzmedium, wie z. B. CGXII-Medium (Keilhauer, C. et al 1993, J. Bacteriol., 175:5593-5603) geeignet. Nach entsprechender Kultivierung kann die Bakteriensuspension geerntet und zur weiteren Untersuchung, beispielsweise zur Transformation oder zur Isolierung von Nukleinsäuren nach gängigen Methoden eingesetzt werden. Diese Vorgehensweise kann analog auch auf andere gram-positive oder gram-negative Bakterienstämme angewendet werden. Dabei werden als Wirtssysteme Bakterien der Gattung Bacillus oder Staphylococcus bevorzugt. Besonders bevorzugt kann hier die Art Bacillus subtilis, Bacillus licheniformis, Bacillus amyloliquefaciens oder Staphylococcus carnosus genannt werden.

Darüber hinaus sind im Rahmen der vorliegenden Erfindung auch Bakterienstämme als Wirtssystem verwendbar, die sich als für die Proteinproduktion geeignete mutierte oder Wild-Typ Stämme auszeichnen, da ihr Stoffwechselfluss verstärkt in Richtung der Biosynthese von Proteinen verläuft.

Ferner sind im Rahmen der vorliegenden Erfindung auch diejenigen Mikroorganismen geeignet, die dem Fachmann aus mikrobiellen Herstellungsverfahren bekannt sind, wie z. B. Enterobacteriaceae oder Corynebacteriaceae.

Als Wirtsorganismen können auch Mikroorganismen eingesetzt werden, bei denen ein oder mehrere Gen(e) codierend für Proteine, Komponenten oder Faktoren, die für den Transport von Proteinen durch die bakterielle Plasmamembran verantwortlich sind, derart verändert sind, dass sie zusätzlich zu dem erfindungsgemäßen SecA Protein zu einem erhöhten Transport von Proteinen durch die Plasmamembran beitragen. Dies können beispielsweise die weiteren bekannten Bestandteile (*secY, secE, secG, secD, secF* und *yajC*) der Sec-Translokase sein. Beispielhaft seien hier Wirtsorganismen der Gattung Bacillus oder Staphylococcus genannt.

Die vorliegende Erfindung wird durch die ausgewählten Beispiele an Mikroorganismen näher charakterisiert, jedoch nicht limitiert.

Die vorliegende Erfindung betrifft ferner einen genetisch veränderten Mikroorganismus enthaltend in replizierbarer Form eine erfindungsgemäße Nukleinsäure der zuvor beschriebenen Art.

Ebenso umfasst die vorliegende Erfindung einen genetisch veränderten Mikroorganismus enthaltend in replizierbarer Form eine erfindungsgemäße Genstruktur oder einen erfindungsgemäßen Vektor der zuvor beschriebenen Art.

Ein erfindungsgemäß genetisch veränderter Mikroorganismus zeichnet sich ferner dadurch aus, dass er ein gram-positives oder gram-negatives Bakterium, wie beispielsweise ein Organismus aus der Familie der Bacillaceae, Staphylococcaceae, Enterobacteriaceae oder Corynebacteriaceae, besonders bevorzugt der Gattung Bacillus oder Staphylococcus ist. Besonders bevorzugt ist beispielsweise Bacillus subtilis, Bacillus licheniformis, Bacillus amyloliquefaciens oder Staphylococcus carnosus.

Die vorliegende Erfindung betrifft darüber hinaus ein Verfahren zur mikrobiellen Herstellung von Proteinen, wobei wenigstens eine der erfindungsgemäßen Nukleinsäuren in einen Wirtsorganismus übertragen oder dort mit den dem Fachmann bekannten Mitteln erzeugt wird und dort exprimiert wird, dieser genetisch veränderte Mikroorganismus zur mikrobiellen Herstellung von Proteinen eingesetzt wird und das entsprechend gebildete Protein aus dem Kulturmedium isoliert wird.

Die erfindungsgemäß hergestellten genetisch veränderten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) zum Zwecke der Produktion von Proteinen kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Als Kohlenstoffquelle können Zucker und Kohlenhydrate wie z. B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden natriumhaltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzu gegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe z. B. Antibiotika hinzugefügt werden. Um aerobe Bedingungen aufrechtzuerhalten werden Sauerstoff oder sauerstoffhaltige Gasmischungen wie z. B. Luft in die Kultur eingetragen. Die Temperatur der Kultur liegt normalerweise bei 30°C bis 38°C und vorzugsweise bei 37°C. Die Kultur wird so lange fortgesetzt, bis sich ein Maximum des gewünschten Proteins gebildet hat. Dieses Ziel wird normalerweise innerhalb von 8 bis 72 Stunden erreicht.

Die Analyse der Proteine kann durch Aktivitätsbestimmung, Sequenzierung oder Elektrophorese erfolgen.

Die Mikroorganismen, die Gegenstand der vorliegenden Erfindung sind, können Proteine beispielsweise aus Glucose, Saccharose, Lactose, Mannose, Fructose, Maltose, Melasse, Stärke, Cellulose oder aus Glycerin und Ethanol herstellen. Es kann sich um die zuvor bereits näher beschriebenen Vertreter der gram-positiven oder gramnegativen Bakterien handeln. Hierbei zeichnen sich die erfindungsgemäß genetisch veränderten Mikroorganismen durch eine erhöhte Protein-Sekretion gegenüber den entsprechend nicht veränderten Mikroorganismen (Wild Typen) oder den Mikroorganismen aus, die lediglich den Vektor ohne Gen-Insert enthalten. In einer besonderen Ausführungsvariante der vorliegenden Erfindung ist gezeigt, dass die Expression des erfindungsgemäßen *secA* Gens im homologen Bacillus subtilis System (d.h. alle Komponenten des Sec-Transportapparats stammen aus Bacillus subtilis) zu einer wenigstens 3-fachen Steigerung der Protein-Akkumulation im Medium im Vergleich zu den Kontrollstämmen führt. Durch Überexpression weiterer Gene, die positiv auf den allgemeinen Stoffwechsel der Protein-Biosynthese wirken, ist eine weitere Steigerung der Protein-Produktion zu erwarten.

Mit dem erfindungsgemäßen Verfahren können Pharmaproteine, Hormone, Enzyme, Wachstumsfaktoren oder beispielsweise Cytokine hergestellt werden. So können beispielsweise Proteasen, Amylasen, Carbohydrasen, Lipasen, Epimerasen, Tautomerasen, Mutasen, Transferasen, Kinasen oder Phosphatasen mikrobiell hergestellt werden.

Die Figuren zeigen beispielhaft verwendete Plasmide, eine schematische Übersicht über den Sec-Proteinsekretionsapparat, eine Darstellung der SecA Proteine mit den hierin beschriebenen Mutationen sowie einen experimentellen Nachweis der erhöhten Proteinsekretion unter Verwendung der hierin beschriebenen Nukleinsäuren.

Es zeigt:
Fig. 1:
Sec-Proteinsekretionsapparat gram-positiver Bakterien
Fig. 2:
Schematische Darstellung der SecA Proteine von *S.* carnosus und *B. subtilis.* Eingezeichnet sind die durch Selektion erhaltenen supprimierenden Mutationen des *S. carnosus* SecA. Die durch Pfeile markierten Mutationen wurden durch ortsgerichtete Mutagenese einzeln auf die korrespondierenden Aminosäurepositionen vom plasmidkodierten *B. subtilis* SecA übertragen.
Fig. 3:
Nachweis von PhoB bzw. PhoB L15Q mittels Western Blot in Zellextrakten und Kulturüberständen von *Bacillus* subtilis DB 104 Stammes bei Co-Expression von plasmidkodiertem PhoB bzw. PhoB L15Q und mutierten SecA Proteinen. Die In-duktion der PhoB Expression erfolgte mit 0,5 mM IPTG, die SecA Expression wurde mit 0,2 % Xylose induziert. **p:** PhoB L15Q Vorläufer; **m:** reifes PhoB (signifikant erhöhte Mengen sind mit Pfeil gekennzeichnet); **L15Q:** Variante der alkalischen Phosphatase PhoB, die aufgrund eines Aminosäureaustausches von Leucin zu Glutamin an Position 15 in der Signalsequenz nur sehr ineffizient im unveränderten *Bacillus subtilis* exexportiert wird; **pWA - secA:** Leervektor; **pWA + secA:** Vektor mit Wild Typ SecA; **pWAX:** Plasmid mit Mutation des *secA*. von *Bacillus subtilis*, X steht für eine der Mutationen in Position 464, 468, 487, 531 bzw. 729

| **Spur** | **Versuchsansatz** |
|---|---|
| 1 | Zellextrakt; Plasmid pWA - *secA*, PhoB unverändert |
| 2 | Überstand; Plasmid pWA - *secA*, PhoB unverändert |
| 3 | Zellextrakt; Plasmid pWA - *secA*, L15Q |
| 4 | Überstand; Plasmid pWA - *secA*; L15Q |
| 5 | Zellextrakt; Plasmid pWA + *secA*; PhoB unverändert |
| 6 | Überstand; Plasmid pWa + *secA*; PhoB unverändert |
| 7 | Zellextrakt; Plasmid pWA + *secA*; L15Q |
| 8 | Überstand; Plasmid pWA + *secA*; L15Q |
| 9 | Zellextrakt; Plasmid pWA464; L15Q |
| 10 | Überstand; Plasmid pWA464; L15Q |
| 11 | Zellextrakt; Plasmid pWA468; L15Q |
| 12 | Überstand; Plasmid pWA468; L15Q |
| 13 | Zellextrakt; Plasmid pWA487; L15Q |
| 14 | Überstand; Plasmid pWA487; L15Q |
| 15 | Zellextrakt; Plasmid pWA531; L15Q |
| 16 | Überstand; Plasmid pWA531; L15Q |
| 17 | Zellextrakt; Plasmid pWA729; L15Q |
| 18 | Überstand; Plasmid pWA729; L15Q |

Fig. 4:
Plasmidvektor pDEL6;
   - secAS.c.:: *secA*-Gen von Staphylococcus carnosus;
   - cat:: Chloramphenicol-Resistenzgen;
   - orf1 und orf3:: Bereiche, die im Chromosom von Bacillus subtilis vor (orf1) bzw. hinter (orf3) dem *secA* Gen liegen. Diese Bereiche die- nen dazu, das secA Gen im Chromosom von Bacillus subtilis gegen das *secA* Gen von Staphylococcus carnosus auszutauschen.
   - bla:: Gensequenz codierend für β-Lactamase

Fig. 5:
Plasmidvektor pCU3seq; (entspricht dem pEF1 Plasmid aus
der Veröffentlichung [19])
   - cat:: Chloramphenicol-Resistenzgen
   - P25/O:: Bakteriophagen T5 Promotor PN₂₅/lac Operator
   - lacI:: Gen kodierend für lac-Repressor
   - Pv:: B. subtilis vegII Promotor
   - bla:: β-Lactamase

Fig. 6:
Plasmidvektor pWH1520
- bla:: β-Lactamase
- tet:: Tetracyclin Resistenzgen
- xylR:: Repressorgen des Xylose Operons aus Bacillus megaterium
- xylA':: N-terminales Fragment der Xylose Isomerase unter Kontrolle eines Xylose induzierbaren Promotors

Fig. 7:
Plasmidvektor pXR100;

- bla:: β-Lactamase
- xylR :: Repressorgen des Xylose Operons aus Bacillus megaterium
- PxylA:: Xylole induzierbarer Promotor der Xylose Iso- merase
- cat:: Chloramphenicol- Resistenzgen

### Ausführungsbeispiele:

### Allgemeine Beschreibung:

Zunächst wurde ein Bacillus subtilis Stamm mit einer künstlich erhöhten Qualitätskontrolle des SecA Proteins, (im folgenden RMA = replacement mutant SecA genannt), konstruiert.

Dieser Stamm wurde erzeugt, indem das auf dem Chromosom liegende Bacillus subtilis *secA*-Gen gegen das *secA*-Gen von Staphylococcus carnosus ausgetauscht wurde. Dieser Austausch resultiert in einer künstlich erhöhten Qualitätskontrolle der Sec-Translokase. Als Modellprotein wurde das heterologe Protein OmpA aus E. coli eingesetzt. Die künstlich erhöhte Qualitätskontrolle des SecA Proteins führte dazu, dass spezifisch das heterologe Protein OmpA von *E. coli* vom Export ausgeschlossen wird. Daneben zeigt die RMA ein kältesensitives Wachstum (d.h. kein Wachstum bei 25°C) sowie Defekte in der Sporenbildung und der Ausbildung von natürlicher Kompetenz.

Im Folgenden wurden Suppressormutanten der RMA selektioniert, die wieder bei 25°C wachsen können und/oder die wieder die Fähigkeit zur Sporenbildung besitzen. Die Charakterisierung dieser Suppressormutanten ergab, dass in diesen Mutanten Veränderungen in dem fremden *secA* Gen aufgetreten waren. Die entsprechenden Aminosäureveränderungen betrafen vor allem die beiden Bereiche IRA-1 und IRA-2, die an der Regulation der ATP-Hydrolyse an der NBS-1 von SecA beteiligt sind. Die weitere Charakterisierung der Suppressormutanten zeigte darüber hinaus, dass das in der RMA vom Export fast vollständig ausgeschlossene heterologe OmpA Protein in den Suppressormutanten deutlich besser exportiert werden kann. Aufgrund des Vergleichs mit *Escherichia coli* SecA Mutanten aus Literaturdaten [10], die zum Teil mit den hierin beschriebenen Mutationen im *secA* Gen bzw. den hierin beschriebenen Veränderungen in der Aminosäuresequenz des SecA Proteins an den entsprechenden Positionen identisch waren, kann angenommen werden, dass die in den IRA Elementen vom Staphylococcus carnosus SecA identifizierten Mutationen die Repression der ATP Hydrolyse an der NBS-1 in SecA abschwächen, wodurch die basale ATPase Aktivität erhöht wird [9, 10]. Die veränderten Staphylococcus carnosus SecA Varianten stellen somit SecA Proteine dar, die einen besseren Export von normalerweise ineffizient oder gar nicht exportierten heterologen Proteinen erlauben.

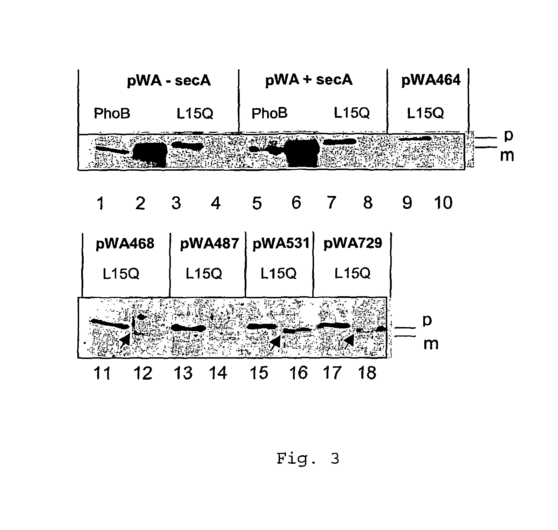

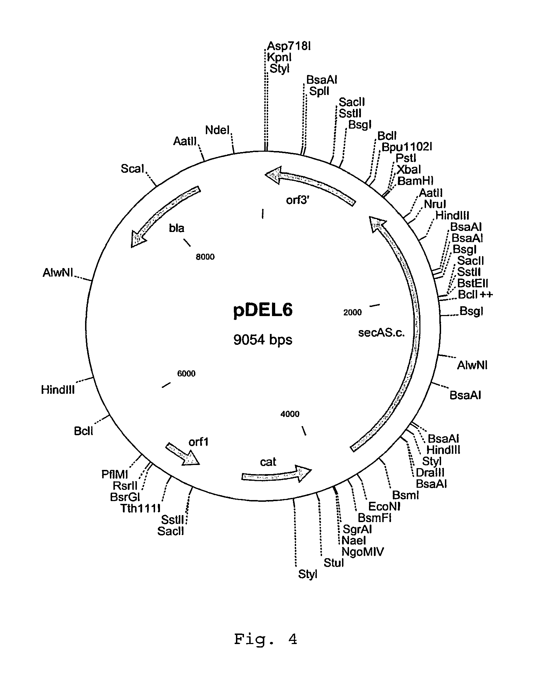

Da die RMA und die isolierten Suppressormutanten alle noch das fremde *secA*-Gen von Staphylococcus carnosus besitzen und somit nach wie vor eine künstliche Situation mit einer gemischten Sec-Translokase darstellen, wurden im Folgenden die gefundenen Veränderungen (einzeln und auch in Kombination) auf das homologe Bacillus subtilis SecA übertragen. Die Coexpression der mutierten Bacillus subtilis SecA-Proteine mit verschiedenen ineffizient transportierten Exportproteinen in Bacillus subtilis zeigte, dass auch im nun homologen System (d.h. alle Komponenten des Sec-Transportapparats stammen aus B. subtilis) ein verbesserter Export der untersuchten Proteine zu beobachten war (gezeigt in Fig.3 am Beispiel einer ineffizient exportierten Variante der alkalischen Phosphatase PhoB von B subtilis).

### 1. Konstruktion der Bacillus subtilis SecA-Austauschmutante (RMA)

Das pDEL6 Plasmid, welches zur Konstruktion der RMA verwendet wurde, wurde wie folgt konstruiert:

Ein pOrf3 Plasmid wurde durch Ligation eines 0,5 kb Asp718/Pst1 Fragmentes vom Plasmid pMKL4 [11] in das Plasmid pGEM3Z (Promega) erhalten. Ein 2,4 kb Nsi1/Pst1 Fragment vom Plasmid pBO1 [12] wurde in das pOrf3 Plasmid ligiert, welches mit Pst1 verdaut wurde. Das *secA* Gen des daraus resultierenden Plasmids pDEL1 wurde durch Verdau mit Spe1 und Sal1 gegen ein 1,4 kb E-coR1/Sal1 Fragment vom pDG268 Plasmid [13], welches das Gen für die Chloramphenicol Acetyltransferase (*cat*) trägt, ausgetauscht, woraus das Plasmid pDEL3 resultierte. Anschließen wurden verschiedene *secA* Gene in das mit Sal1 und Pst1 verdaute Plasmid pDEL3 ligiert. Das pDEL5 Plasmid trägt das *B. subtilis secA* Gen, welches durch Sal1/Pst1 Verdau vom pMKL4 Plasmid [11] als 2,6 kb Fragment erhalten wurde. Das pDEL6 Plasmid trägt das *S. carnosus secA* Gen, welches durch Sac1/Pst1 Verdau vom pMA12 Plasmid [14] als 2,6 kb Fragment erhalten wurde. Das pDEL6 Plasmid, welches zur Konstruktion der RMA eingesetzt wurde, enthält damit Bereiche aus fünf verschiedenen Plasmiden:
- *secA*-Gen aus dem Plasmid pMA12
- Chloramphenicol-Resistenzgen (cat) aus dem Plasmid pDG268
- orf1 aus Plasmid pBO4
- orf3 aus Plasmid pMKL4
- Restlicher Bereich (lacZ und bla) aus Plasmid pGEM3Z Die Bacillus subtilis SecA-Austauschmutante (RMA) wurde erhalten, indem der Wildtypstamm *B. subtilis* DB104 *(his, nprR2, nprE18, ΔaprA3)* [15] mit dem linearisierten Plasmid pDEL6 transformiert wurde und Klone selektioniert wurden, die in Gegenwart von 10 µg/ml Chloramphenicol wachsen konnten. Der *secA* Genaustausch wurde durch Southern-Blot Analysen verifiziert, wobei das *B. subtilis* bzw. das *S*. *carnosus secA* Gen als Sonde verwendet wurde.

### 2. Isolierung von Suppressormutanten der B. subtilis RMA

Die *B. subtilis* RMA wurde als Parentalstamm verwendet, um Suppressormutanten zu isolieren, die wieder bei 25 °C wachsen können, bzw. besser sporulieren können als die RMA.

Zur Isolierung von kälteresistenten Suppressormutanten der RMA wurde Luria-Bertani (LB) Medium nach Zugabe von 10 µg/ml Chloramphenicol mit 10⁶ Zellen der RMA beimpft und 48 Stunden bei 25°C inkubiert. Es wurden zwischen 100 und 1000 Zellen auf LB-Agarplatten ausplattiert und weitere 24 Stunden bei 25°C inkubiert. Alternativ wurden 10⁶ Zellen aus einer Kultur der RMA, die über Nacht bei 37°C inkubiert wurde, direkt auf LB-Agarplatten ausplattiert und 24 Stunden bei 25°C inkubiert. Die Stabilität der Mutation der so erhaltenen Suppressormutanten wurde überprüft, indem die Mutanten nach Überimpfen und Inkubation bei 37°C auch nach erneutem Überimpfen und Inkubation bei 25°C weiterhin Wachstum zeigten.

Zur Isolierung von Suppressormutanten, die wieder besser sporulieren können als die RMA, wurde 5 ml Sporulationsmedium [16] 1:100 mit einer gewaschenen Übernachtkultur der RMA beimpft und mindestens 24 Stunden bei 37 °C inkubiert. 100 µl des Ansatzes wurden nach Zugabe der gleichen Menge Chloroform 30 Minuten bei 80°C erhitzt. Die Sporen wurden auf LB-Agarplatten ausplattiert und 2 Tage bei 25°C inkubiert. Mutanten, die unter diesen Selektionsbedingungen wachsen konnten, wurden isoliert.

### 3. Klonierung und Sequenzierung der secA Gene der Suppressormutanten

*B. subtilis* DB 104 wurde verwendet, um zu unterscheiden, ob die supprimierenden Mutationen der Suppressormutanten im *secA* Gen von *S. carnosus* oder einem anderen Gen von *B. subtilis* lokalisiert sind. Hierzu wurde *B*. *subtilis* DB 104 mit chromosomaler DNA der Suppressormutanten transformiert und Chloramphenicol resistente Klone selektioniert. Die so erhaltenen Transformanten wurden getestet, ob sie auch bei 25°C wachsen können, somit außer dem Gen für die Chloramphenicol Acetyltransferase auch das benachbarte, stromabwärts liegende *S*. *carnosus secA* Gen mit der supprimierenden Mutation anstelle des *B*. *subtilis secA* Gens in das Chromosom integriert hatten. Es wurde festgestellt, dass die supprimierenden Mutationen der Suppressormutanten im *secA* Gen von *S*. *carnosus* lokalisiert sind. Daher wurde anschließend eine Klonierung der *secA* Gene durchgeführt.

Um die *S. carnosus secA* Gene der Suppressormutanten unter der Kontrolle des Xylose induzierbaren Promotors *xylA* zu klonieren, erfolgte eine Amplifizierung von *se*cA mittels PCR unter Verwendung chromosomaler DNA der Suppressormutanten als Matrize. Hierfür wurden die Primer

SecAS.c.NBamH1 (5' CGGGATCCCAAAGGAGCGAACAGAATGGG 3') SecAS.c.CSph1 (5' ACATGCATGCATACAACTTACTATTTACCGCAGC 3') verwendet (unterstrichene Basen zeigen die DamH1 bzw. Sph1 Schnittstelle an). Nach Amplifikation wurde ein 2,56 kb Fragment erhalten, welches mit BamH1 und Sph1 verdaut wurde und in den ebenfalls BamH1/Sph1 verdauten Vektor pWH1520 [17] ligiert wurde, wodurch die Plasmide pWH*secAS.c.*ₛᵤₚₚ. erhalten wurden. Die Sequenzierung der *secA Gene* erfolgte mit IRD800 markierten Primern
SecAS.c. H1 (5' AACTGCAACGATGCCGAC 3'),
SecAS.c.H2 (5' GTGCTGATAAAGCTGAACG 3')
SecA S.c.H3 (5' AATTCCAACGAACCGTCC 3'),
SecAS.c.H4 (5' GACAAGGTGACCGCGGAG 3'),
SecAS.c.H5 (5' AAGGTAAAGATCGTGAGG 3') und
SecAS.c.R5 (5' CTGTTCAAGTTCAATCCG 3') (MWG) unter Verwendung des Thermo Sequenase fluorescent labelled primer cycle sequencing Kit (Amersham Pharmacia Biotech) nach Angaben des Herstellers.

### 4. Transfer supprimierender Mutationen in das SecA von B. subtilis

Die Übertragung supprimierender Mutationen im SecA von *S. carnosus* auf die entsprechenden Aminosäurereste im SecA von *B. subtilis* erfolgte mittels ortsgerichteter Mutagenese von plasmidkodiertem *B. subtilis* SecA unter Verwendung des QuikChange Site-Directed Mutagenesis Kit von Stratagene nach Angaben des Herstellers. Als Matrize für die PCR Reaktion wurde das Plasmid pMKL40 und folgende Primerpaare zum Einfügen der gewünschten Mutationen verwendet (die veränderten Basen sind unterstrichen) :
1. Austausch von Histidin zu Glutamin an Aminosäureposition 464
   5' GTGTTAAATGCCAAAAACCAAGAACGTGAAGCGCAGATC 3'
   5' GATCTGCGCTTCACGTTCTTGGTTTTTGGCATTTAACAC 3'
2. Austausch von Alanin zu Valin an Position 468
   5' CCATGAACGTGAAGTTCAGATCATTGAAGAGGCCGGCC 3'
   5' GGCCGGCCTCTTCAATGATCTGAACTTCACGTTCATGG 3'
3. Austausch von Alanin zu Valin an Position 487
   5' CGATTGCGACTAACATGGTTGGGCGCGGAACGG 3'
   5' CCGTTCCGCGCCCAACCATGTTAGTCGCAATCG 3'
4. Austausch von Asparaginsäure zu Alanin an Position 531
   5' CCGGACGTCAGGGAGCCCCGGGGATTACTC 3'
   5' GAGTAATCCCCGGGGCTCCCTGACGTCCGG 3'
5. Austausch von Asparaginsäure zu Valin an Position 729
   5' GGATGGATCATATTGTTGCGATGGATCAGCTCCGCCAAGGG 3'
   5' CCCTTGGCGGAGCTGATCCATCGCAACAATATGATCCATCC 3'

Ein 1,86 kb SnaB1/Sph1 *secA* Fragment aus pMKL40, in welchem die gewünschte Mutation vorhanden war, wurde anschließend mit dem 8,67 kb Fragment des mit SnaB1 und Sph1 verdauten Vektors pWMKL1 ligiert. Daraus entstanden die Plasmide pWAX (X steht für eines der Konstrukte 464, 468, 487, 531 bzw. 729), welche die *secA* Gene mit den gewünschten Mutationen in einem B*. subtilis* Expressionsvektor unter der Kontrolle des Xylose induzierbaren Promotors pxylA enthielten. Die Sequenzierung der veränderten *B. subtilis secA* Gene erfolgte mit den Primern
SecAB.s.H1 (5' GTACAGCTAAGACAGAGG 3'),
SecAB.s.H2 (5' TTGACCGCTTCGGCATGG 3'),
SecAB.s.H3 (5' AAGGGATTCACCTTCGTGC 3'),
SecAB.s.R1 (5' TTTCCTTCCATCGTGCGG 3'),
SecAB.s.R2 (5' TTCAGTAAGCTGTACAGC 3') und
SecAB.s.R3 (5' TTTCCGTC ATGAAGCGCC 3').

Die Expression der SecA Proteine wurde überprüft, indem LB Medium nach Zugabe von 0,2 % (w/v) Xylose mit den Stämmen *B*. *subtilis* DB104, welche die Plasmide pWAₓ enthielten, zu einer OD₆₀₀ von 0,4 angeimpft wurde und anschließend 4 Stunden bei 37°C inkubiert wurde. Zellen aus 2 ml Kultur wurden abzentrifugiert und nach Aufschluß mit 50 µl Lysispuffer (10 mM Tris/HCl pH 8,0, 25 mM MgCl₂, 200 mM NaCl, 5 mg/ml Lysozym) in dem gleichen Volumen 2 x Laemmli Probenpuffer aufgekocht. Die Proteine wurden in einem 10 % SDS Polyacrylamidgel aufgetrennt und das SecA mittels Western-Blot Analyse unter Verwendung von *B. subtilis* SecA spezifischen Antikörpern nachgewiesen. Die Funktionalität der veränderten *B*. *subtilis* SecA Proteine wurde überprüft, indem die temperatursensitive *B*. *subtilis secA* Mutante NIG1152 *(met, his, div341^{ts})* [18] mit den Plasmiden pWAX transformiert wurde und durch Induktion der SecA Expression mit 0,2 % (w/v) Xylose der Wachstumsdefekt der Mutante bei der nicht-permissiven Temperatur von 42 °C komplementiert wurde.

### 5. Untersuchung des Proteinexports einer alkalischen Phosphatase PhoB

Es wurde untersucht, ob durch Expression der veränderten *B. subtilis* SecA Proteine der Proteinexport im *B*. *subtilis* Wildtyp verbessert wird. Als Exportsubstrat wurde eine Variante der alkalischen Phosphatase PhoB verwendet, die aufgrund eines Aminosäureaustausches von Leucin zu Glutamin an Position 15 (PhoBL15Q) in der Signalsequenz nur sehr ineffizient im *B. subtilis* Wild-typ exportiert wird. B. subtilis DB104 wurde mit den Plasmiden pCU3phoB bzw. pCU3phoBL15Q, welche die Gene für das Wildtyp PhoB bzw. die Variante PhoBL15Q enthalten, sowie den Plasmiden pWAₓ., welche die veränderten secA *B. subtilis* Gene enthalten, transformiert. Mit den so erhaltenen Stämmen wurde LB Medium, mit 0,5 mM IPTG und 0,2 % Xylose versetzt, zu einer OD₆₀₀ von 0,4 angeimpft und die Kulturen 4 Stunden bei 37°C inkubiert. 2 ml Zellen wurden durch Zentrifugation vom Überstand getrennt, mit 50 µl Lysispuffer aufgeschlossen und in dem gleichen Volumen 2x Laemmli Probenpuffer aufgekocht.

Proteine aus den Überständen wurden über Nacht mit 13 % Trichloressigsäure bei 4 °C gefällt, zweimal mit 80 % Azeton gewaschen und in 50 µl Laemmli Probenpuffer aufgekocht. Von den Zellextrakten wurde ein Volumen entsprechend 0,2 OD Zellen und von den Überständen ein Volumen entsprechend 1,0 OD Zellen auf ein 12,5 % SDS Polyacrylamidgel aufgetragen und das PhoB mittels PhoB spezifischer Antikörper im Western-Blot nachgewiesen. Die Ergebnisse zeigt Fig. 3.

### Literatur

1. Harwood C.R. (1992): Bacillus subtilis and its relatives: molecular biological and industrial workhorses. Trends Biotechnol. 10: 247-256
2. Simonen M., Palva I. (1993): Protein secretion in Bacillus species. Microbiol. Rev. 57: 109- 137
3. Freudl, R. (1992) Protein secretion in Gram-positive bacteria. J. Biotechnol. 23: 231-240.
4. Freudl, R. (1998) Proteinsekretion bei Gram-positiven Bakterien: Molekulare Grundlagen und biotechnologische Aspekte. Biospektrum 4 (no. 1): 29-33.
5. van Wely, Karel H. M.; Swaving, Jelto; Freudl, Roland, Driessen, Arnold J. M. (2001) Translocation of proteins across the cell envelope of Gram-positive bacteria. FEMS Microbiol. Rev. 25: 437-454.
6. Fekkes, P.; Driessen, A. J. M. (1999) Protein targeting to the bacterial cytoplasmic membrane. Microbiology and Molecular Biology Reviews 63: 161-173.
7. Nakatogawa, H., Mori, H., und Ito, K. (2000) Two independent mechanisms down-regulate the intrinsic SecA ATPase activity. J. Biol. Chem. 275: 33209-33212.
8. Sianidis, G., Karamanou, S., Vrontou, E., Boulias, K., Repanas, K., Kyrpides, N., Politou, AS., und Economou, A. (2001) Cross-talk between catalytic and regulatory elements in a DEAD motor domain is essential für SecA function. EMBOJ. 20: 961-970.
9. Baud, C., Karamanou, S., Sianidis, G., Vrontou, E., Politou, A. and Economou, A. (2002) Allosteric communication between signal peptides and the SecA protein DEAD Motor ATPase domain. J. Biol. Chem. 277: 13724-13731.
10. Matsumoto, G., Nakatogawa, H., Mori, H., und Ito, K. (2000) Genetic dissection of SecA: suppressor mutations against the secY translocase defect. Genes to Cells 5: 991-999.
11. Klose, M., Schimz, K.L., van der Wolk, J., Driessen, A.J.M., Freudl, R. (1993) Lysine 106 of the putative ATP-binding site of Bacillus subtilis SecA protein is required for functional complementation of Escherichia coli secA mutants in vivo. J. Biol. Chem. 268: 4504-4510
12. Overhoff, B., Klein, M., Spies, M., Freudl, R. (1991) Identification of a gene fragment which codes for the 364 amino-terminal amino acid residues of a SecA homologue from Bacillus subtilis: further evidence for the conservation of the protein export apparatus in gram-positive and gram-negative bacteria. Mol. Gen. Genet. 228: 417-423
13. Antoniewski, C., Savelli, B., Stragier, P. (1990) The spoIIJ gene, which regulates early developmental steps in Bacillus subtilis belongs to a class of environmentally responsive genes. J. Bacteriol. 172: 86-93
14.Klein, M., Meens J., Freudl R. (1995) Functional characterization of the Staphylococcus carnosus SecA protein in Escherichia coli and Bacillus subtilis secA mutant strains. FEMS Microbiol. Lett. 131: 271-277
15. Kawamura, F., Doi, R.H. (1984) Construction of a Bacillus subtilis double mutant deficient in extracellular alkaline and neutral protease. J. Bacteriol. 160: 442-444
16.Schaeffer, P., Millet, J., Auber, J.P. (1965) Catabolic repression of bacterial sporulation. Proc. Natl. Acad. Sci. USA 54: 704-711
17. Rygus, T., Hillen, W. (1991) Inducible high-level expression of heterolgous genes in Bacillus megaterium using the regulatory elements of the xyloseutilization operon. Appl. Microbiol. Biotechnol. 35: 594-599
18.Miyakawa, Y., Komano, T. (1981) Study of the cell cycle of Bacillus subtilis using temperaturesensitive mutants. Isolation and genetic analysis of the mutants defective in septum formation. Mol. Gen. Genet. 181: 207-214
19. Meens, J., Frings, E., Klose, M., und Freudl, R. (1993) An outer membrane protein (OmpA) of Escherichia coli can be translocated across the cytoplasmic membrane of Bacillus subtilis. Mol. Microbiol. 9: 847-855

## Patentansprüche

1. SecA Protein aus *Staphylococcus carnosus* mit einer Aminosäuresequenz gemäß SEQ ID NO: 2, welches mindestens einen Aminosäureaustausch ausgewählt aus der Gruppe bestehend aus dem Aminosäureaustausch von Tyrosin zu Histidin an Position 198, dem Aminosäureaustausch von Valin zu Glutaminsäure an Position 665 und dem Aminosäureaustausch von Asparaginsäure zu Valin an Position 734 aufweist.

2. SecA Protein aus *Bacillus subtilis* mit einer Aminosäuresequenz gemäß SEQ ID NO: 4, welches den Aminosäureaustausch von Asparaginsäure zu Valin an Position 729 aufweist.

3. Nukleinsäure, enthaltend ein *secA* Gen aus *Staphylococcus carnosus* gemäß SEQ ID NO: 1, welche mindestens eine Mutation ausgewählt aus der Gruppe bestehend aus der Mutation von T zu C an Position 592, der Mutation von T zu A an Position 1994 und der Mutation von A zu T an Position 2210 aufweist.

4. Nukleinsäure, enthaltend ein *secA* Gen aus *Bacillus subtilis* gemäß SEQ ID NO: 3, welche die Mutation von A zu T an Position 2186 aufweist.

5. Genstruktur, enthaltend wenigstens eine Nukleinsäure nach Anspruch 3 oder 4 sowie mit diesen operativ verknüpfte regulatorische Sequenzen.

6. Vektor, enthaltend wenigstens eine Nukleinsäure nach Anspruch 3 oder 4 oder eine Genstruktur nach Anspruch 5 sowie zusätzliche Nukleotidsequenzen zur Selektion, zur Replikation in einer Wirtszelle oder zur Integration in das Wirtszellgenom.

7. Mikroorganismus, enthaltend wenigstens eine Nukleinsäure nach Anspruch 3 oder 4, eine Genstruktur nach Anspruch 5 oder einen Vektor nach Anspruch 6 in replizierbarer Form.

8. Mikroorganismus nach Anspruch 7, wobei der Mikroorganismus ein grampositives oder gramnegatives Bakterium ist.

9. Mikroorganismus nach Anspruch 8, wobei das Bakterium zur Familie der *Bacillaceae, Staphylococcaceae, Enterobacteriaceae* oder *Corynebacteriaceae* gehört.

10. Mikroorganismus nach Anspruch 9, wobei das Bakterium zur Gattung *Bacillus, Staphylococcus, Escherichia* oder *Corynebacterium* gehört.

11. Mikroorganismus nach Anspruch 10, wobei das Bakterium aus der Gruppe bestehend aus *Bacillus subtilis, Bacillus licheniformis, Bacillus amyloliquefaciens, Staphylococcus carnosus, Escherichia coli* oder *Corynebacterium glutamicum* ausgewählt ist.

12. Verfahren zur mikrobiellen Herstellung von Proteinen, **dadurch gekennzeichnet, dass**
a) wenigstens eine Nukleinsäure gemäß Anspruch 3 oder 4 in einen Mikroorganismus übertragen oder in dem Mikroorganismus erzeugt wird, und die Nukleinsäure in dem Mikroorganismus exprimiert wird,
b) der genetisch veränderte Mikroorganismus aus Schritt a) zur mikrobiellen Herstellung von Proteinen eingesetzt wird, und
c) das in Schritt b) hergestellte Protein aus einem Kulturmedium isoliert wird.

13. Verfahren nach Anspruch 12, **dadurch gekennzeichnet, dass** das in Schritt b) hergestellte Protein ein homologes oder heterologes Protein ist.

14. Verfahren nach Anspruch 12 oder 13, **dadurch gekennzeichnet, dass** das in Schritt b) hergestellte Protein ein Hormon, Enzym, Wachstumsfaktor, Pharmaprotein oder Cytokin ist.

15. Verfahren nach Anspruch 14, **dadurch gekennzeichnet, dass** das Enzym eine Protease, Amylase, Carbohydrase, Lipase, Epimerase, Tautomerase, Mutase, Transferase, Kinase oder Phosphatase ist.

## Claims

1. A SecA protein of *Staphylococcus carnosus* having an amino acid sequence according to SEQ ID NO: 2, which has at least one amino acid change selected from the group consisting of the amino acid change of tyrosine into histidine in position 198, the amino acid change of valine into glutamic acid in position 665 and the amino acid change of aspartic acid into valine in position 734.

2. A SecA protein of *Bacillus subtilis* having an amino acid sequence according to SEQ ID NO: 4, which has an amino acid change of aspartic acid into valine in position 729.

3. A nucleic acid, containing a *secA* gene of *Staphylococcus carnosus* according to SEQ ID NO: 1, which has at least one mutation selected from the group consisting of the mutation of T into C in position 592, the mutation of T into A in position 1994 and the mutation of A into T in position 2210.

4. A nucleic acid, containing a *secA* gene of *Bacillus subtilis* according to SEQ ID NO: 3, which has the mutation of A into T in position 2186.

5. A gene structure, containing at least one nucleic acid of claim 3 or 4 and regulatory sequences that are operationally linked to said nucleic acids.

6. A vector, containing at least one nucleic acid of claim 3 or 4 or a gene structure of claim 5 and additional nucleotide sequences for selection, replication in a host cell or integration into the host cell genome.

7. A microorganism, containing at least one nucleic acid of claim 3 or 4, a gene structure of claim 5 or a vector of claim 6 in replicable form.

8. The microorganism of claim 7, wherein the microorganism is a Gram-positive or Gram-negative bacterium.

9. The microorganism of claim 8, wherein the bacterium belongs to the family *Bacillaceae, Staphylococcaceae, Enterobacteriaceae* or *Corynebacteriaceae.*

10. The microorganism of claim 9, wherein the bacterium belongs to the genus *Bacillus, Staphylococcus, Escherichia* or *Corynebacterium.*

11. The microorganism of claim 10, wherein the bacterium is selected from the group consisting *of Bacillus subtilis, Bacillus licheniformis, Bacillus amyloliquefaciens, Staphylococcus carnosus, Escherichia coli* or *Corynebacterium glutamicum.*

12. A method for microbial production of proteins, **characterized in that**
a) at least one nucleic acid according to claim 3 or 4 is transferred in a microorganism or is produced in the microorganism, and the nucleic acid is expressed in the microorganism,
b) the genetically modified microorganism of step a) is used for the microbial production of proteins, and
c) the protein produced in step b) is isolated from the culture medium.

13. The method of claim 12, **characterized in that** the protein produced in step b) is a homologous or heterologous protein.

14. The method of claim 12 or 13, **characterized in that** the protein produced in step b) is a hormone, enzyme, growth factor, pharmaprotein or cytokine.

15. The method of claim 14, **characterized in that** the enzyme is a protease, amylase, carbohydrase, lipase, epimerase, tautomerase, mutase, transferase, kinase or phosphatase.

## Revendications

1. Protéine SecA de *Staphylococcus carnosus* comprenant une séquence d'acides aminés selon la séquence SEQ ID NO: 2, laquelle protéine présente au moins une substitution d'acides aminés sélectionnée parmi le groupe consistant en la substitution d'acides aminés de tyrosine en histidine en position 198, en la substitution d'acides aminés de valine en acide glutaminique en position 665 et en la substitution d'acides aminés d'acide asparaginique en valine en position 734.

2. Protéine SecA de *Bacillus subtilis* comprenant une séquence d'acides aminés selon la séquence SEQ ID NO: 4, laquelle protéine présente la substitution d'acides aminées d'acide asparaginique en valine en position 729.

3. Acide nucléique contenant un gène *secA* de *Staphylococcus carnosus selon* la séquence SEQ ID NO: 1, lequel acide présente au moins une mutation sélectionnée parmi le groupe consistant en la mutation de T en C en position 592, en la mutation de T en A en position 1994 et en la mutation de A en T en position 2210.

4. Acide nucléique contenant un gène *secA* de *Bacillus subtilis* selon la séquence SEQ ID NO: 3, lequel acide présente la mutation de A en T en position 2186.

5. Structure génétique contenant au moins un acide nucléique selon la revendication 3 ou 4 ainsi que des séquences régulatrices reliées opérationnellement à celui-ci.

6. Vecteur contenant au moins un acide nucléique selon la revendication 3 ou 4 ou une structure génétique selon la revendication 5 ainsi que des séquences nucléotidiques supplémentaires pour la sélection, la réplication dans une cellule hôte ou pour l'intégration dans le génome de la cellule hôte.

7. Micro-organisme contenant au moins un acide nucléique selon la revendication 3 ou 4, une structure génétique selon la revendication 5 ou un vecteur selon la revendication 6 dans une forme pouvant être répliquée.

8. Micro-organisme selon la revendication 7, ce micro-organisme étant une bactérie à Gram positif ou une bactérie à Gram négatif.

9. Micro-organisme selon la revendication 8, la bactérie appartenant à la famille des *Sacillaceae, Staphylococcaceae, Enterobacteriaceae ou Corynebacteriaceae.*

10. Micro-organisme selon la revendication 9, la bactérie appartenant à l'espèce *Bacillus, Staphylococcus, Escherichia ou Corynebacterium.*

11. Micro-organisme selon la revendication 10, la bactérie étant sélectionnée parmi le groupe consistant en les *Bacillus subtilis, Bacillus licheniformis, Bacillus amyloliquefaciens, Staphylococcus carnosus, Escherichia coli ou Corynebacterium glutamicum.*

12. Procédé pour la production bactériologique de protéines, **caractérisé en ce que**
a) au moins un acide nucléique selon la revendication 3 ou 4 est transféré dans un micro-organisme ou produit dans le micro-organisme, et l'acide nucléique s'exprime dans le micro-organisme.
b) le micro-organisme génétiquement modifié et issu de l'étape a) est utilisé pour la production bactériologique de protéines, et
c) la protéine produite à l'étape b) est isolée à partir d'un milieu de culture.

13. Procédé selon la revendication 12, **caractérisé en ce que** la protéine produite à l'étape b) est une protéine homologue ou hétérologue.

14. Procédé selon la revendication 12 ou 13, **caractérisé en ce que** la protéine produite à l'étape b) est une hormone, un enzyme, un facteur de croissance, une protéine pharmaceutique ou une cytokine.

15. Procédé selon la revendication 14, **caractérisé en ce que** l'enzyme est une protéase, amylase, carbohydrase, lipase, épimérase, tautomérase, mutase, transférase, kinase ou phosphatase.
